# EUROPÄISCHE PATENTANMELDUNG

(11) **EP 0 152 944 A2**
(43) Veröffentlichungstag der Anmeldung: **28.08.1985**
(21) Anmeldenummer: 85101806.9
(22) Anmeldetag: 20.02.1985
(51) Int. Cl.: C07H 19/16, A61K 31/70

(54) **Verwendung von Adenosin-Derivaten als Antiallergica und Arzneimittel, die diese enthalten**

(30) Priorität: 23.02.1984 DE 3406533
(71) Anmelder: BOEHRINGER MANNHEIM GMBH, 68298 Mannheim (DE)
(72) Erfinder: Schaumann, Wolfgang, Prof. Dr. med., D-6900 Heidelberg (DE); Wilhelms, Otto-Henning, Dr. rer.nat., D-6941 Weinheim-Ritttenweier (DE); Roesch, Andronika, Dr. med., D-6800 Mannheim 1 (DE); Kampe, Wolfgang, Dr. rer.nat., D-6805 Heddesheim (DE)

(57) **Zusammenfassung**

Verwendung von Adenosin-Derivaten der Formel die eine Hemmung der SRS-A-Freisetzung aufweisen, 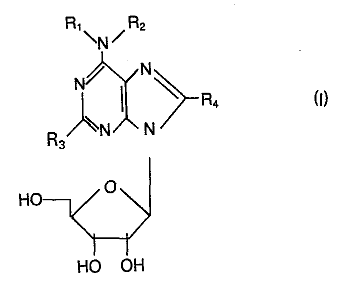 zur Behandlung von allergischen Erkrankungen sowie entzünjungsbedingten, bronchospastischen und bronchokonstriktorischen Reaktionen, allein oder zusammen mit einem Xanthin-Derivat der Formel II 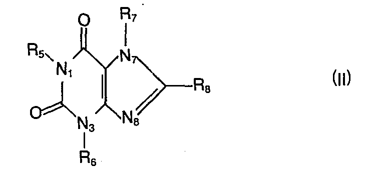 und Arzneimittel, die neben üblichen Trägerstoffen als Wirkkomponente eine Verbindung der Formel und eine Verbindung der Formel enthalten.

## Beschreibung

Es ist bekannt, daß Adenosin und zahlreiche seiner Derivate eine Vielzahl von pharmakologischen Wirkungen besitzen. Neben der Erweiterung der Gefäße, Blutdrucksenkung, Bradykardie, AV-Block und einer Hemmung der Lipolyse sind auch Wirkungen auf das Immunsystem beschrieben. Weiterhin ist bekannt, daß Adenosin und ein N-monosubstituiertes Derivat, das Phenylisopropyladenosin (L-PIA), die durch Antigen ausgelöste Freisetzung von Histamin und anderen Mediatoren wie SRS-A (slow reacting substance of anaphylaxis) aus isolierten Mastzellen und Lungengewebe verstärken. Nach derzeitigem Stand des Wissens wirken deshalb Adenosin und L-PIA pro-allergisch.

Es war deshalb außerordentlich überraschend, daß andere, wegen ihrer gefäßerweiternden, blutdrucksenkenden und lipolysehemmenden Eigenschaften bereits untersuchten Adenosin-Derivate in vitro und in vivo eine antiallergische Wirkung zeigen. Dies läßt sich relativ einfach durch Untersuchungen zur Antiqen-bedinqten SRS-A-Freisetzung aus Lungengewebeproben bestimmen.

Gegenstand der vorliegenden Erfindung ist deshalb die Verwendung von Adenosinderivaten, die eine Hemmung der SRS-A-Freisetzung aufweisen zur Behandlung allergischer Krankheiten sowie von entzündungsbedingten bronchospastischen und bronchokonstriktorischen Reaktionen.

Die erfindungsgemäß wirksamen Verbindungen umfassen Adenosin-Derivate der folgenden allgemeinen Formel I 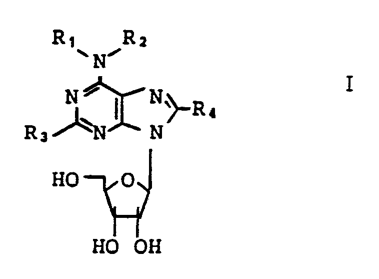 in welcher
R₁ und R₂ gleich oder verschieden sind und eine geradkettige oder verzweigte Alkylgruppe, die ein- oder mehrfach durch Hydroxy-, Alkoxycarbonyl-oder Alkoxy-Gruppen,durch Phenalkyl oder Alkyl substituierte Aminogruppen, wobei der Phenylteil gegebenenfalls ebenfalls substituiert sein kann, durch gegebenenfalls ein- oder mehrfach durch Hydroxy, Alkoxy, Alkyl, Trifluormethyl, Halogen, Amino oder Methylsulfonamino substituierte Aryl-, Hetaryl- oder Aryloxy-Gruppen oder durch Cycloalkyl, das mit einem Arylring anelliert sein kann, substituiert sein kann,
eine geradkettige oder verzweigte Alkenylgruppe, eine gesättigte oder ungesättigte Cycloalkylgruppe, die mit einem Arylring anelliert sein kann,

oder gemeinsam mit dem Stickstoffatom, an das sie gebunden sind, einen heterocyclischen Ring bilden können, der gegebenenfalls durch ein weiteres Sauerstoff-, Schwefel- oder Stickstoffatom unterbrochen sein kann, wobei der Stickstoff durch Alkyl oder Aralkyl substituiert sein kann,

R₃ Wasserstoff, Hydroxy, Alkoxy, Halogen, Morpholin, Piperidin oder eine Aminogruppe, die durch Alkyl oder gegebenenfalls substituiertes Aralkyl substituiert sein kann, und

R₄ Wasserstoff, Halogen,Morpholin, Piperidin oder eine gegebenenfalls durch Alkyl oder Aralkyl substituierte Aminogruppe darstellen,

wobei einer der Reste R₁ oder R₂ auch Wasserstoff sein kann, mit der Maßgabe, daß der andere Rest nicht eine Phenylisopropylgruppe sein darf, sowie deren pharmakologisch verträgliche Salze.

Die in der Formel I beschriebenen Verbindungen sind überwiegend bekannt (vgl. DE-OS 16 70 077, 16 70 175, 16 70 265, 21 48 838, 21 57 036, 22 44 328, 23 38 705, 23 38 963, 24 26 682) oder können nach den allgemeinen Methoden hergestellt werden, wie sie in den vorstehenden Druckschriften beschrieben sind. Neue Verbindungen sind insbesondere Verbindungen der Formel I, in denen R₄ Halogen, Morpholin, Piperidin oder eine gegebenenfalls durch Alkyl oder Aralkyl substituierte Aminogruppe darstellt.

Generell sind im Sinne der Erfindung solche Verbindungen der Formel I bevorzugt, die in N(6)-Stellung disubstituiert sind, d.h. R₁ und R₂ nicht Wasserstoff sind.

Bei den oben genannten Aryloxy-, Aryl- oder Hetarylresten der Substituenten R₁ und R₂ handelt es sich um Mono- oder Bicyclen. Unter Aryl ist insbesondere Phenyl- und Naphthyl zu verstehen. Ein Aralkylsubstituent in der Bedeutung von R₁, R₂, R₃ und R₄ ist vorzugsweise ein Benzyl- oder Phenylisopropylrest.

Unter Cycloalkylresten der Substituenten R₁ und R₂ sind Reste mit 3 - 7 Kohlenstoffatomen zu verstehen, insbesondere der Cyclopropyl, Cyclopentyl-, Cyclohexyl- oder Cycloheptyl-Rest. Als ungesättigter Rest kommt vorzugsweise der Cyclohexenyl-oder Cycloheptenyl-Rest infrage.

Die Cycloalkylreste können mit einem Arylrest, vorzugsweise einem Phenylrest anelliert sein. Bevorzugt sind Indan, Tetralin oder der Benzocyclobutan -Ring.Als Hetarylreste kommen vorzugsweise der Furanyl-, Thiophenyl- oder Pyridyl-Rest infrage. Alle diese vorstehend genannten Aryl- bzw. Hetarylreste können unsubstituiert sein oder einen oder mehrere der folgenden Substituenten tragen: Alkyl, Alkenyl, Alkoxy, Alkylthio, Trifluormethyl, Hydroxy, Halogen, Nitro, Aminocarbonyl, Alkoxycarbonyl oder Aminosulfonyl.

Sämtliche genannten Alkyl-Gruppen enthalten 1-10, vorzugsweise 1-8 Kohlenstoffatome, wie z.B. Methyl, Ethyl, n-Propyl, Isopropyl, n-Butyl, Isobutyl, tert.Butyl, Pentyl-, Hexyl oder 1,5-Dimethyln-hexyl. Als Substituent von Aryl- oder Hetaryl-Resten kommen vorzugsweise ein Methyl- oder Ethylrest infrage.

Alkoxyreste sind Reste mit 1-6, vorzugsweise 1-4 Kohlenstoffatomen, insbesondere Methoxy, Ethoxy, Isopropoxy oder n-Propoxy.

Alkenylreste weisen 3-8, vorzugsweise 3-6 Kohlenstoffatome auf, bevorzugt ist der A11y1- oder Butenyl-Rest.

Für den Fall, daß R₁ und R₂ gemeinsam mit dem Stickstoffatom an das sie gebunden sind, einen Heterocyclus bilden, sind hierunter Ringsysteme mit 3-8 Kohlenstoffatomen zu verstehen. Bevorzugt ist der Dimethylenimino, Trimethylenimino-, Tetramethylenimino-, Pentamethylenimino- oder Hexamethylenimino-Ring. Diese Ringsysteme können durch ein Sauerstoff-, Schwefel-oder ein zusätzliches Stickstoffatom unterbrochen sein, wobei das Stickstoffatom durch eine niedere Alkylgruppe oder ein Benzylrest substituiert sein kann. Bevorzugte Ringsysteme sind Morpholin, Thiamorpholin und Piperazin.

Unter Halogen sind vorzugsweise Fluor, Chlor oder Brom zu verstehen.

Wirkungsprüfung von Adenosin-Derivaten in vivo und vitro

### Hemmung des Antigen-bedingten Bronchospasmus am Meerschweinchen in vivo

DerAntigen-bedingte Bronchospasmus beim Meerschweinchen ist ein typisches Modell für die durch Antigen ausgelösten Spasmen beim Menschen. Phenylisopropyladenosin (PIA) wurde unter folgenden Versuchsbedingungen mit einer erfingungsge₋ mäßen Verbindung (BM 11.189) in diesem Versuchsmodell am Meerschweinchen verglichen und die Resultate in der Tabelle 1 zusammengefaßt.

Einfluß von BM 11.189 und TH 162 (L-PIA) auf den allergisch bedingten Bronchospasmus am passiv sensibilisierten Meerschweinchen

### Methode:

KONZETT, H. und R. RÖSSLER

Versuchsanordnung zu Untersuchungen an der Bronchialmuskulatur. Naunyn-Schmiedebergs Arch. exp. Path. Pharmak. 195, 71 - 74 (1940).

COLLIER, H.O.J.; J.A. Holgate, M. SCHACHTER:
The Bronchoconstrictor action of Bradykinin in the guinea pig. Brit. J. Pharmacol. 15, 290 (1960).

Antiserumgewinnung und passive Sensibilisierung

Zur Gewinnung von Antiserum wurden männliche Albino-Meerschweinchen sensibilisiert mit einer Emulsion, die zu gleichen Teilen aus kristallisiertem Ovalbumin (5 mg/kg in 0.9 % NaC1-Lösung) und komplettem Freund'schem Adjuvans bestand. Das Antiserum wurde 4 Wochen danach durch Kanülierung der Bauchaorta der mit Penthrane narkotisierten Tiere gewonnen und anschließend bei -18°C gelagert.

24 h vor dem eigentlichen Versuch wurden Meerschweinchen passiv sensibilisiert durch i.v.-Applikation von 1,5 ml Antiserumverdünnung/kg (1:50 verdünnt mit 0,9 %iger NaCI-Lösung).

### Versuchsanordnung zur Messung des Bronchospasmus

Nach der Methode von Konzett-Rössler (1940) in der Modifikation von COLLIER u.a. (1960) wurden passiv sensibilisierte Meerschweinchen in Pentobarbital-Na-Narkose (40 mg/kg i.p.) nach Einbinden einer Kanüle in die Trachea und einer weiteren Kanüle in die V. jugularis an eine Atempumpe angeschlossen. Mit der Atempumpe wurde den Tieren ein Luftvolumen von 7 -15 ml 72 mal/Minute zugeführt. Durch intravenöse Applikation von Ovalbumin (1 mg) wurde der Antigen-bedingte Bronchospasmus hervorgerufen, so daß das Beatmungsvolumen nicht vollständig in die Lunge einströmen konnte und teilweise durch einen Seitenarm über ein Ventil abfloß. Dieses Luftvolumen wurde indirekt als Strömungsgeschwindigkeit mit Hilfe eines Thermoelements gemessen und diente als Maß für die Stärke des 3ronchospasmus. Die Applikation der Prüfsubstanzen erfolgte in die V. jugularis 5 Minuten vor der Antigeninjektion.

Der Substanz-bedingte Einfluß auf den Antigen-induzierten Bronchospasmus wurde ermittelt durch Vergleich von behandelter zu Kontrollgruppe für den Zeitpunkt 3 Minuten nach Antigengabe und die Hemmwirkung in Prozent berechnet.

Die Auswertung wurde nach folgender Formel vorgenommen: 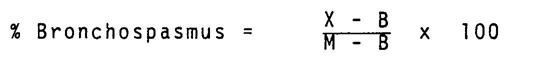
X - B der nach Antigengabe beobachtete in mm Kurvenausschlag gemessene Bronchospasmus
M - B der durch Abklemmen der Trachealkanüle erreichbare maximale Bronchospasmus in mm Kurvenausschlag gemessen
B ist der Ausgangswert in mm Kurvenausschlag

Die Signifikanz dieser Ergebnisse wurde statistisch mit dem t-Test gesichert.

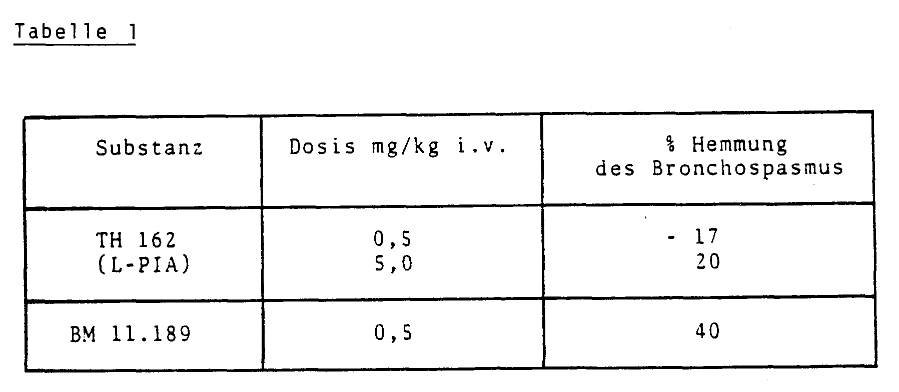

### Ergebnisse:

BM 11.189 bewirkte nach 0.5 mg/kg i.v. eine 40 %ige signifikante Hemmung des Antigen-bedingten Bronchospasmus am passiv sensibilisierten Meerschweinchen.

L-PIA dagegen war bei gleicher Applikationsweise in derselben Versuchsanordnung nach 0.5 und 5.0 mg/kg unwirksam.

B. Hemmung der Antigen-bedingten SRS-A-Freisetzung aus Meerscnweinchen-Lungengewebe in vitro

Für die in vitro Untersuchung der erfindungsgmäßen Verbindungen wurde die Antigen-bedingte SRS-A-Freisetzung aus passiv sensibilisiertem Lungengewebe von Meerschweinchen untersucht. Die Untersuchung wurde nach folgender Methode durchgeführt:
Frisch entnommenes Lungengewebe, das vorher mit Krebs-Puffer, pH 7.4, in situ weitgehend blutfrei gespült worden war, wurde mit einem Mc Ilwain-Gewebezerhacker zerkleinert, mit Krebs- Puffer, pH 7.4, gewaschen und für 1 h bei Raumtemperatur mit einer 1:50-Verdünnung eines homologen anti-Ovalbumin-Antiserums inkubiert. Das Antiserum war vorher in Meerschweinchen erzeugt worden durch wiederholte Injektion von Ovalbumin (2 x kristallisiert) unter Zusatz von komplettem Freund'schen Adjuvans nach DAVIES (15). Bis zu seiner Verwendung wurde das Antiserum bei -18° C unverdünnt gelagert.

Anschließend wurde das passiv sensibilisierte Gewebe zweimal mit Krebspuffer, pH 7,4, gewaschen und Proben von jeweils ca. 400 mg in Krebspuffer mit und ohne Zusatz der Prüfsubstanz (i.A. 3 x 10⁻⁵M)

### (15) DAVIES, G.E., T.P. Johnstone

Quantitative studies on anaphylaxis in guinea pigs passively sensitized with homologous antibody. Inter. Arch. Allergy 41, 648 - 654 (1971)

für 30 min inkubiert, bevor die Antigen-bedingte Freisetzung von SRS-A durch Zusatz von Ovalbumin-Lösung (Endkonzentration: 10 mcg/ml) ausgelöst wurde. Die Freisetzungsreaktion wurde nach 15 min gestoppt durch Zugabe von gleichem Volumen eiskalter Tyrode-Lösung, pH 8,0. Nach Trennung vom Gewebe durch Zentrifugation wurden die Überstände bei -18° C aufgewahrt bis zur Messung ihres SRS-A-Gehalts im Ileum-Bioassay.

Der Ileum-Bioassay wurde mit einem teilautomatisierten Verfahren in Superfusion an Ileumstückchen aus unbehandelten Meerschweinchen durchgeführt. Als Arbeitspuffer wurde Tyrode-Lösung, pH 8.0, mit Zusatz von Atropin (2 x 10-7M) und Mepyramin (10⁻⁶M) eingesetzt.

Die Superfusionszeit für die Probe betrug 3 min, anschließend erfolgte eine Spülphase von 6 min. Als Maß für den Gehalt an SRS-A diente die maximale Kontraktionsamplitude (K). Nach Stimulation mit Ovalbumin stieg der SRS-A-Gehalt in den Proben- Überständen im Vergleich zur Pufferkontrolle im Mittel um Faktor 3 an.

Die Substanz-bedingte Hemmwirkung in Prozent zur "spezifisch" Antigen-induzierten SRS-A-Freisetzung wurde folgendermaßen ermittelt:

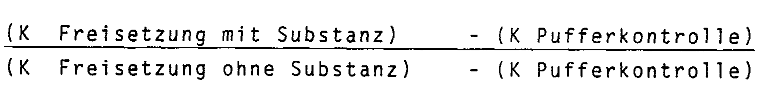

### Ergebnisse

Im oberen Teil der Tab. 2 sind die Ergebnisse mit L-PIA und Adenosin zusammengestellt, die in literaturbekannter Weise die Antigen-bedingte Freisetzung von SRS-A fördern. Die Förderung der SRS-A-Freisetzung war im Konzentrationsbereich von 10⁻⁵-10⁻⁷M annähernd gleich stark.

BM 11.189, welches eingehender geprüft wurde, und zahlreiche andere Adenosin-Derivate zeigten eine mehr oder weniger deutliche Hemmung der SRS-A-Freisetzung (s. Tabelle 2).

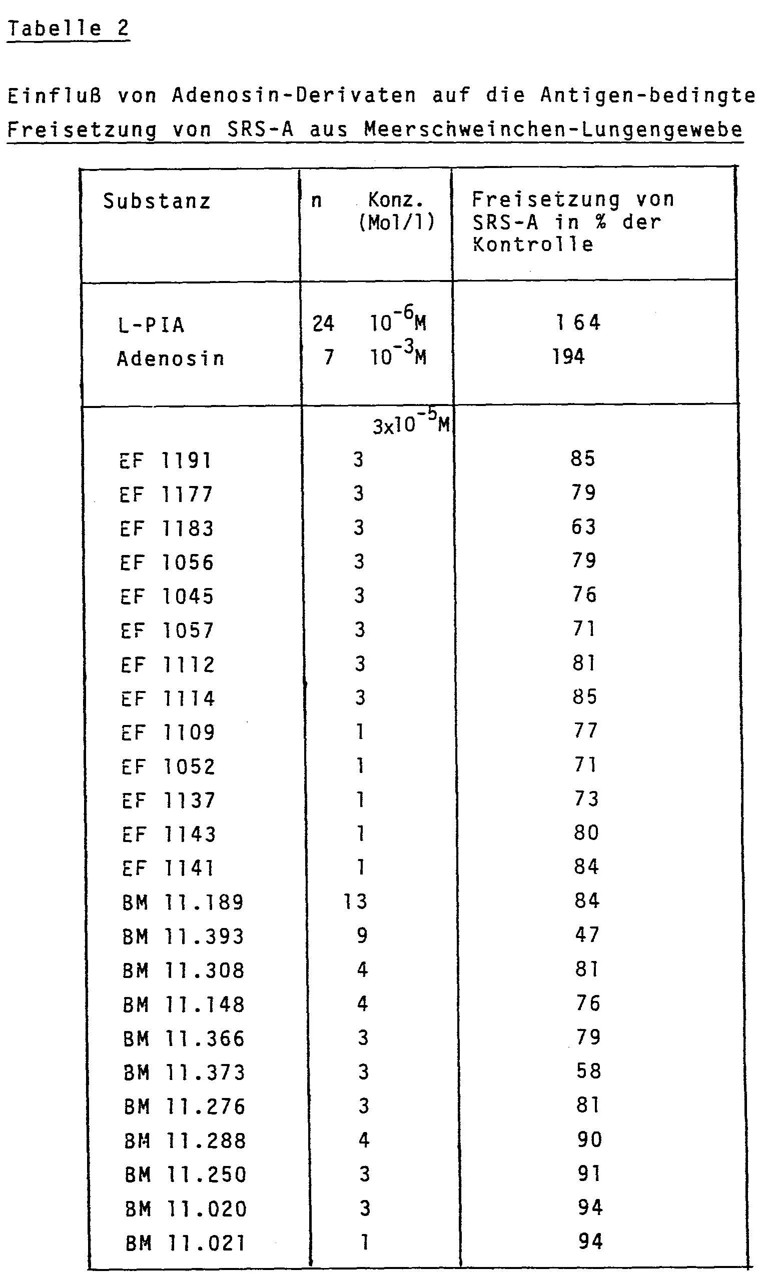

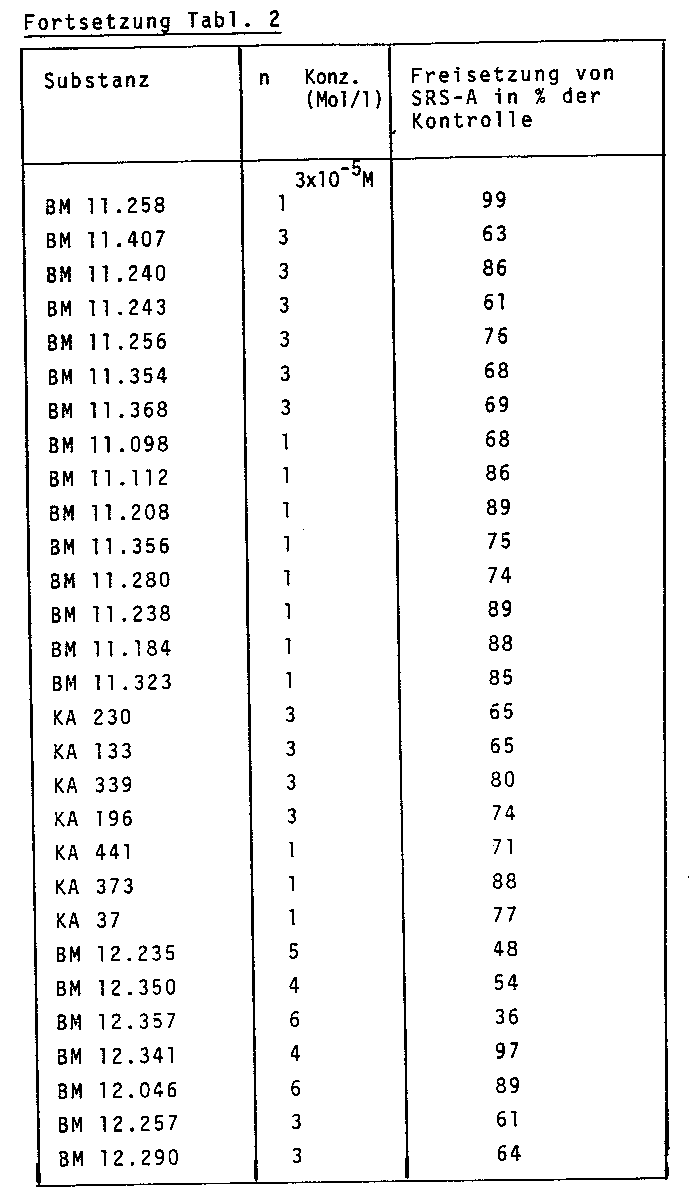

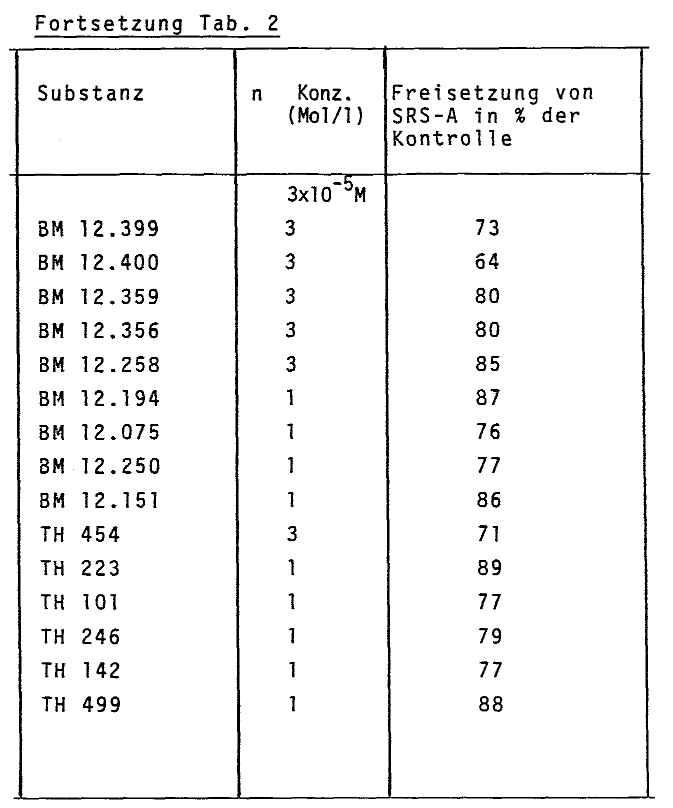

Eine Zusammenstellung der getesteten Verbindungen gemäß Formel I ist in Tabelle 3 zu finden.

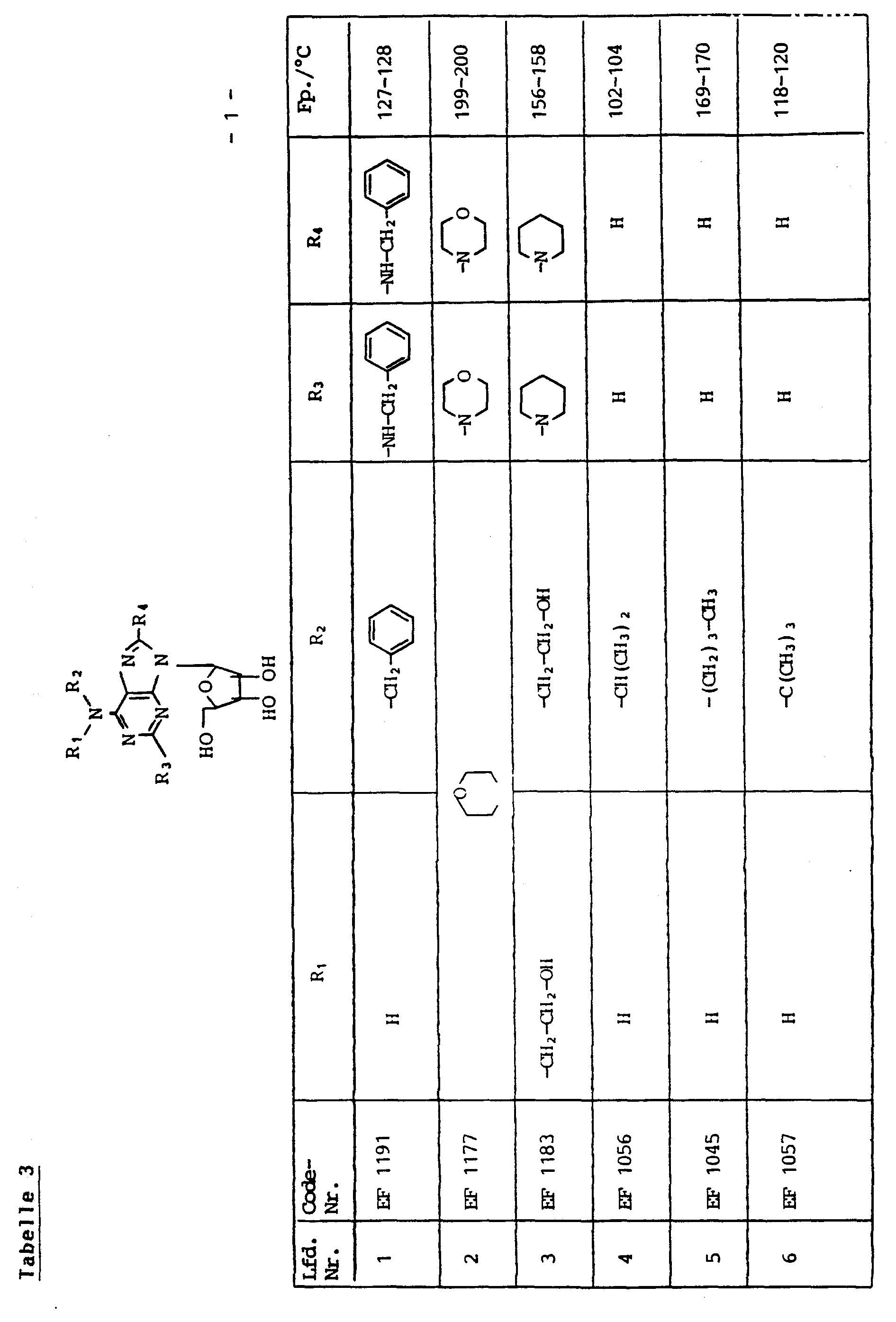

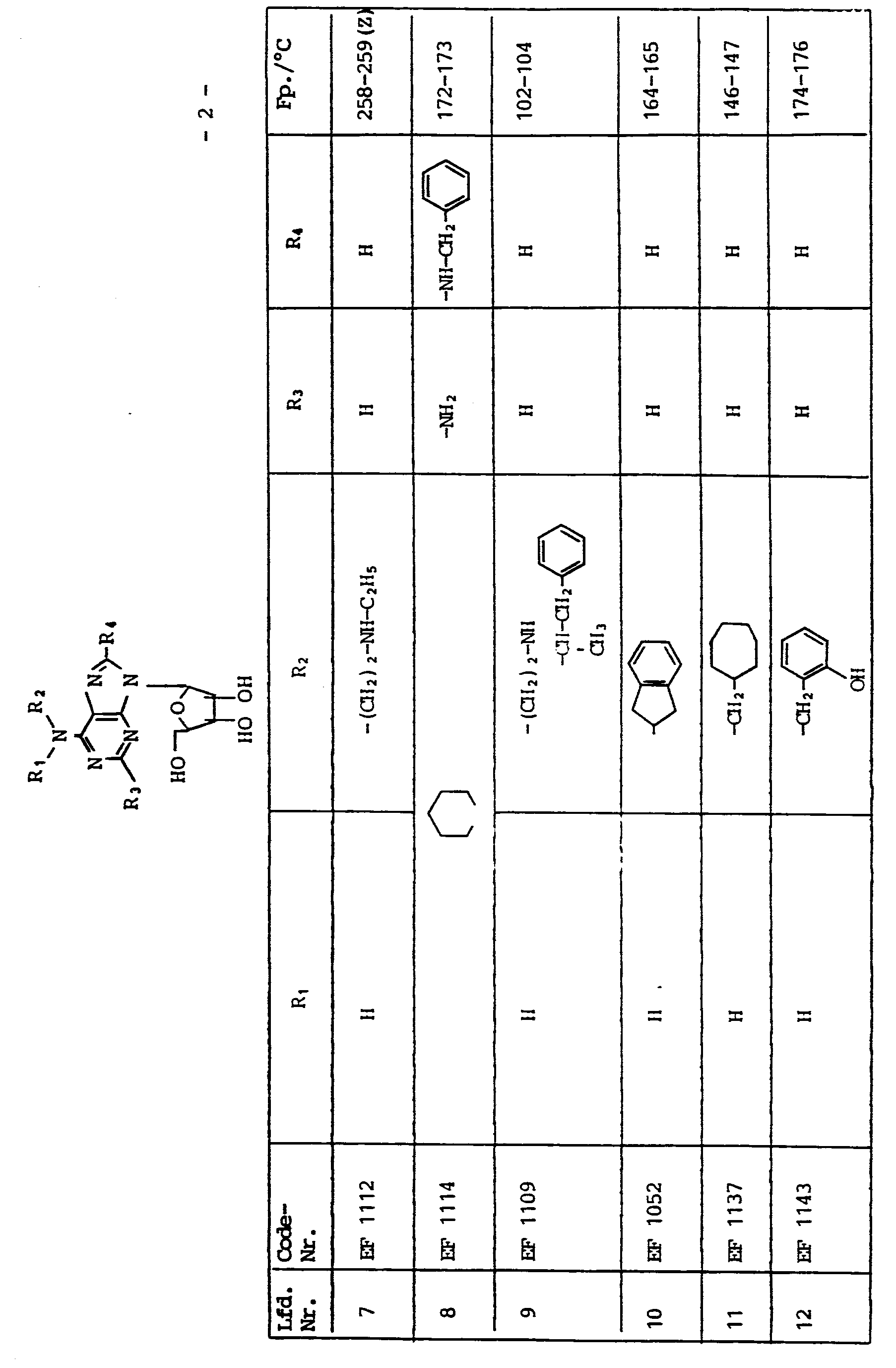

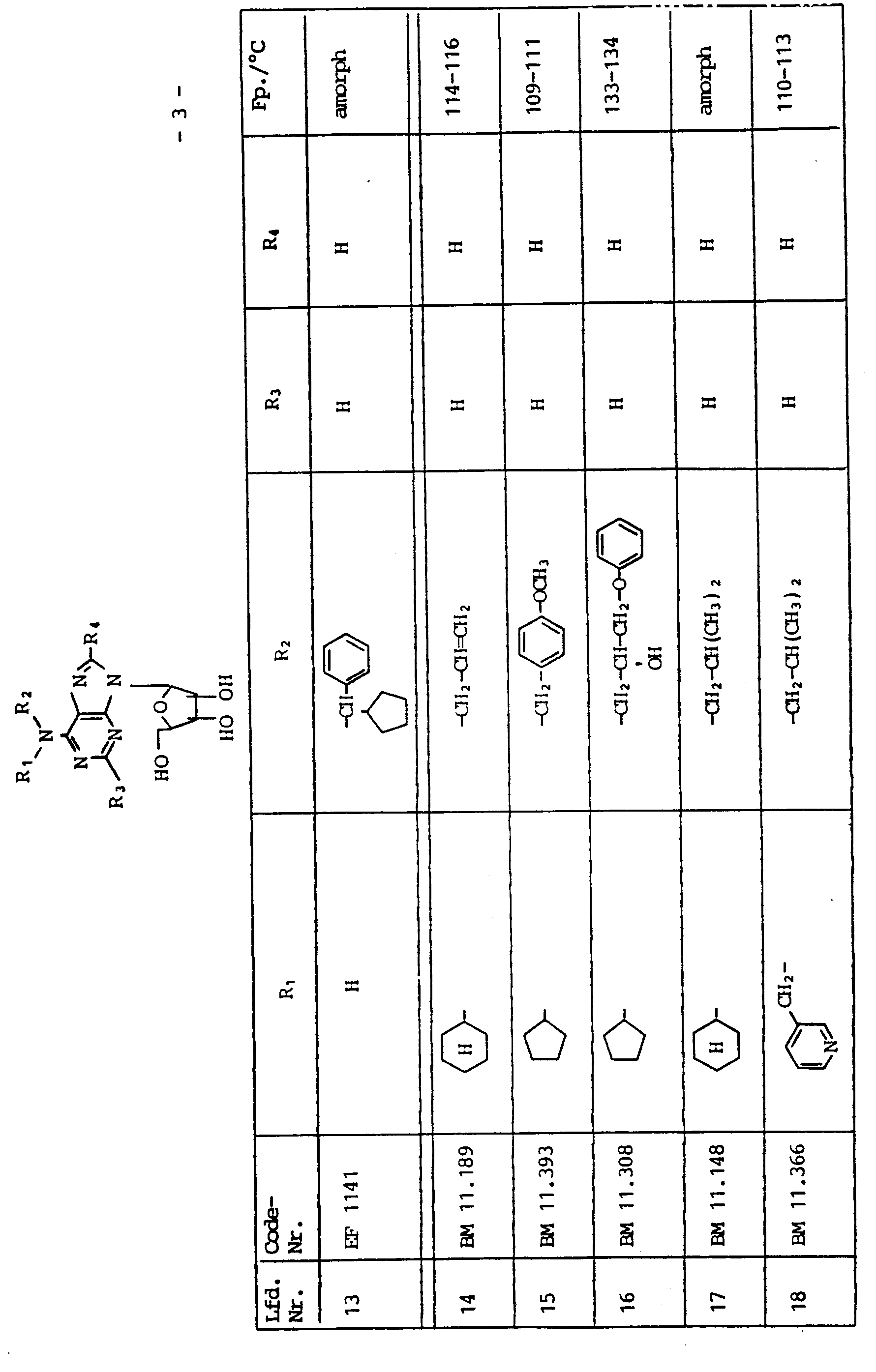

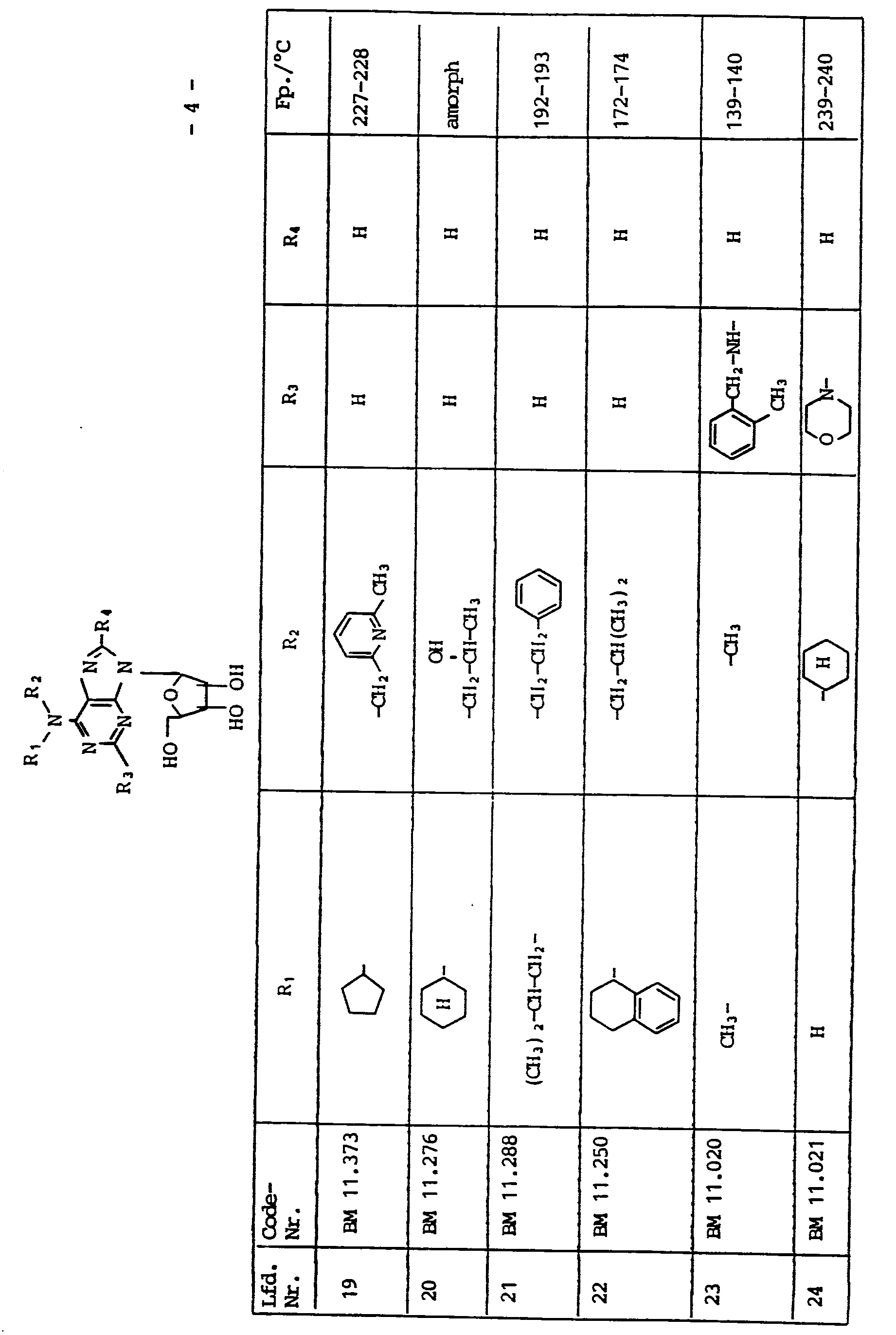

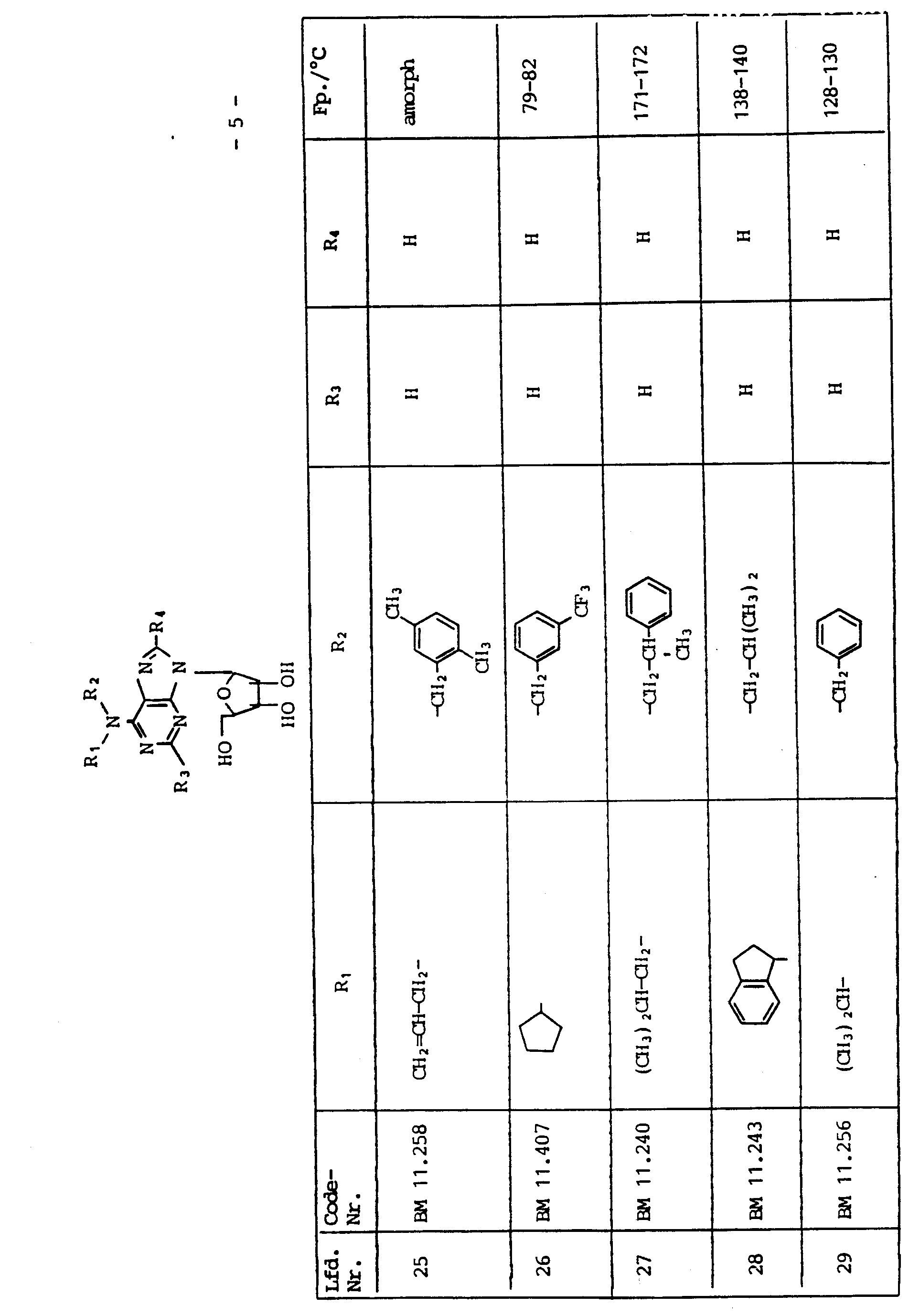

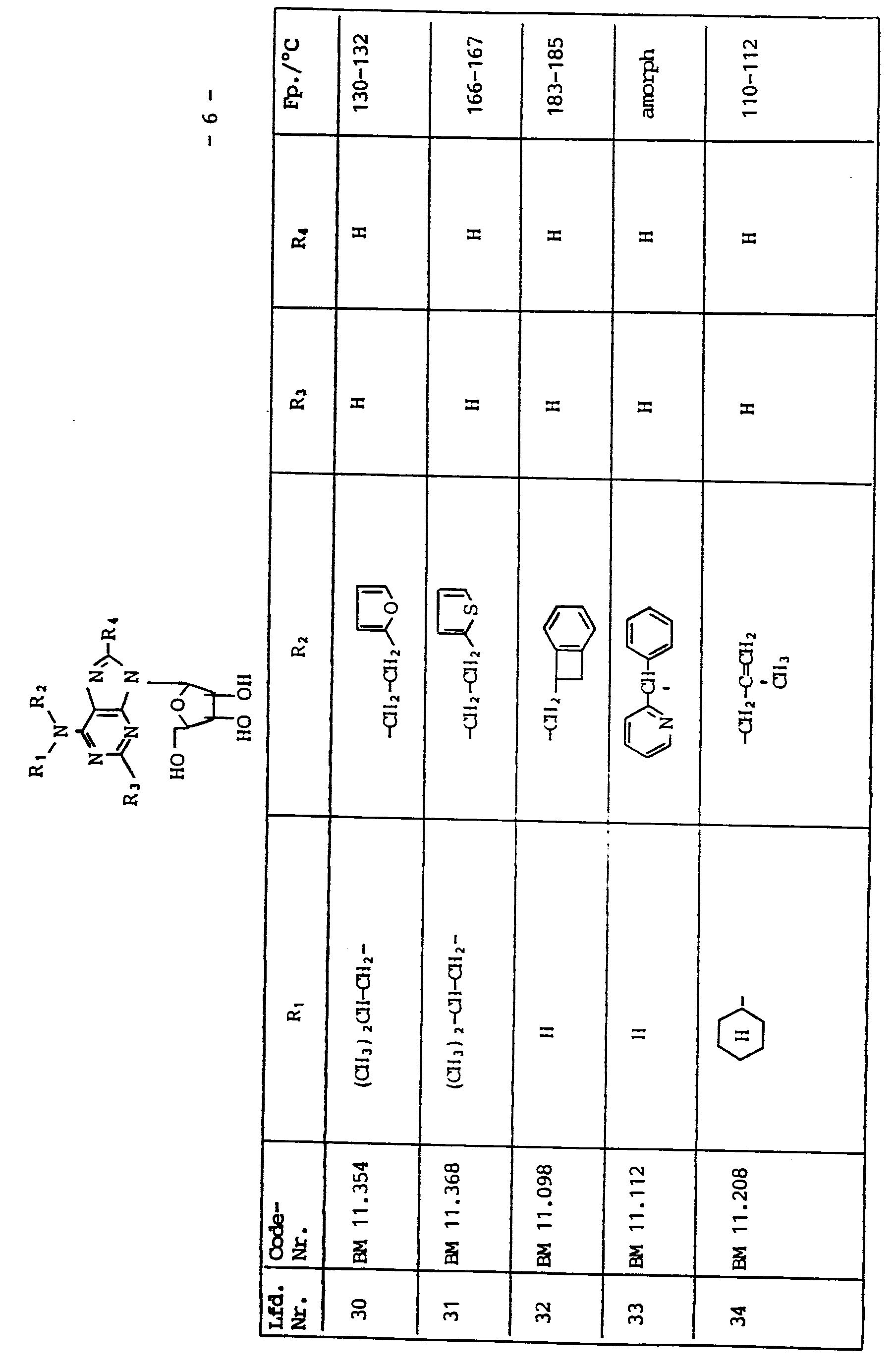

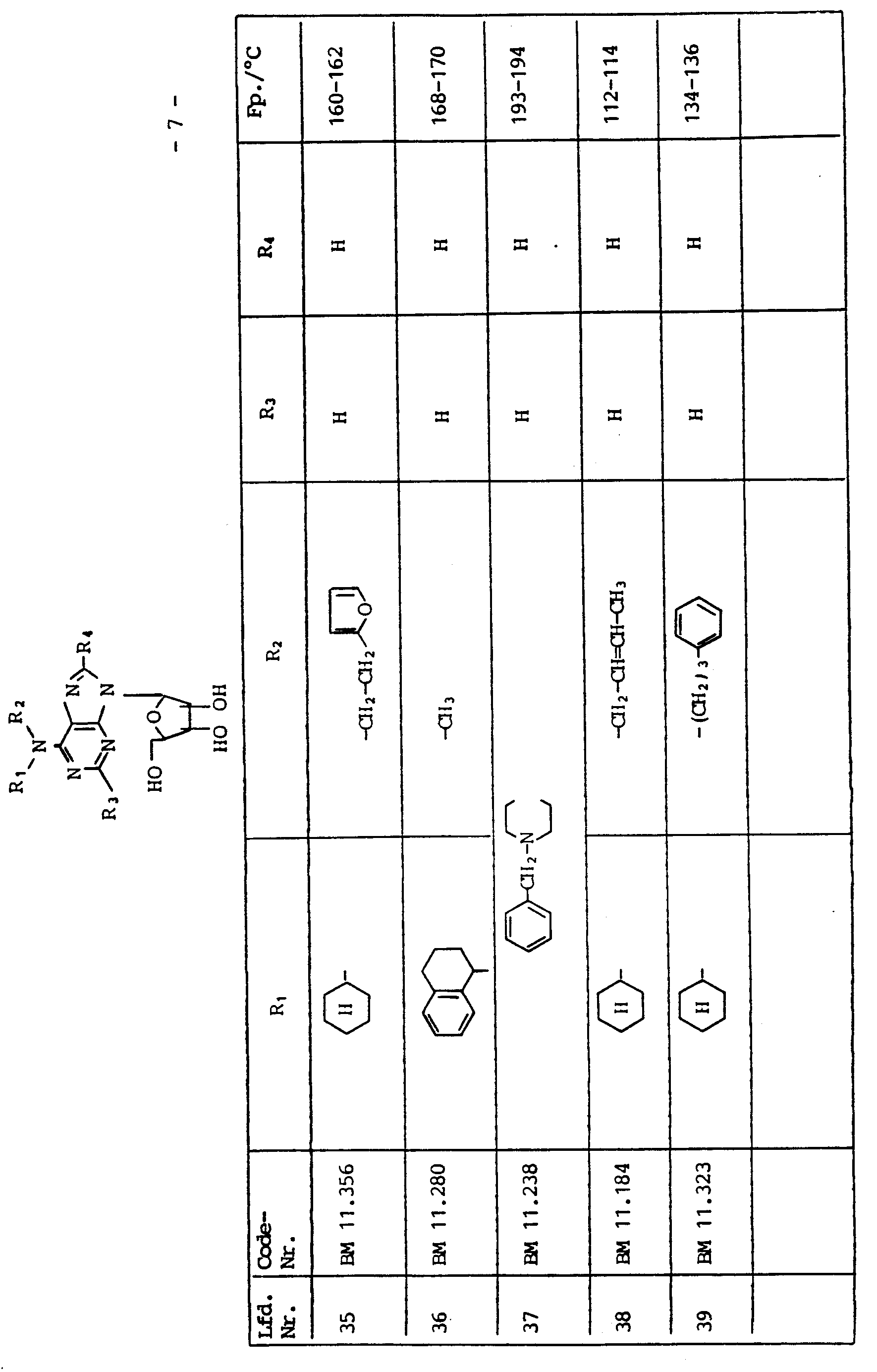

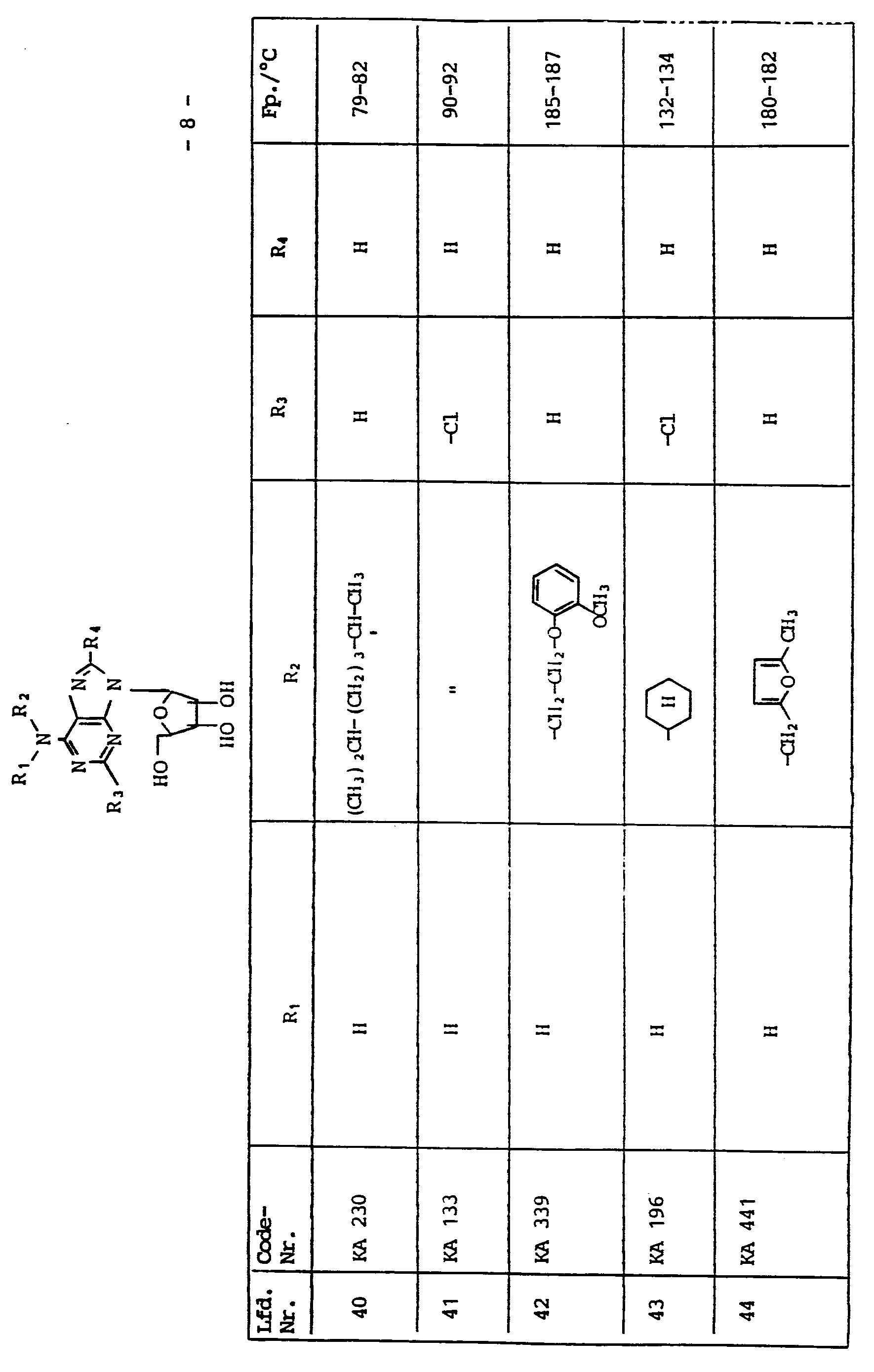

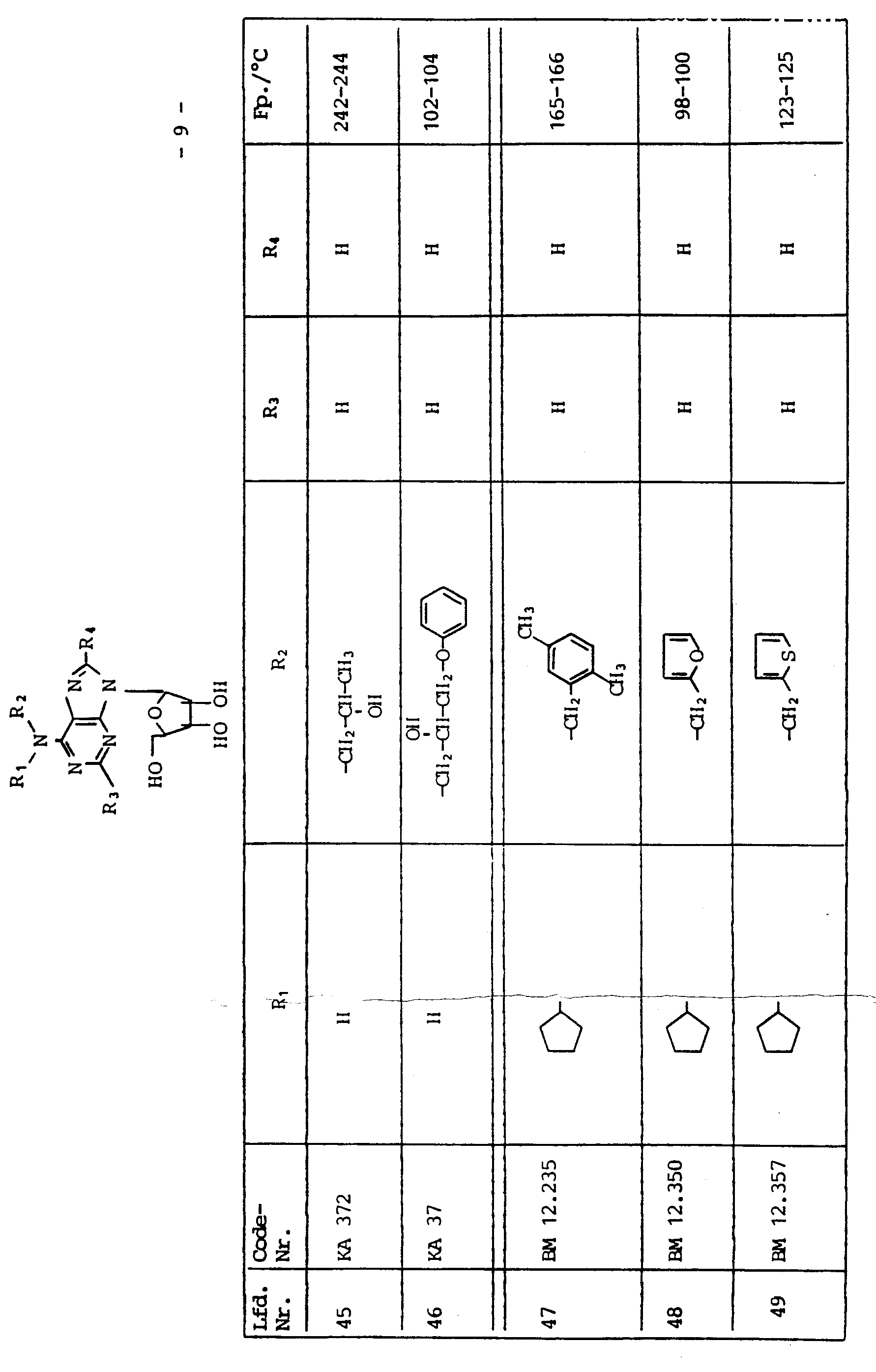

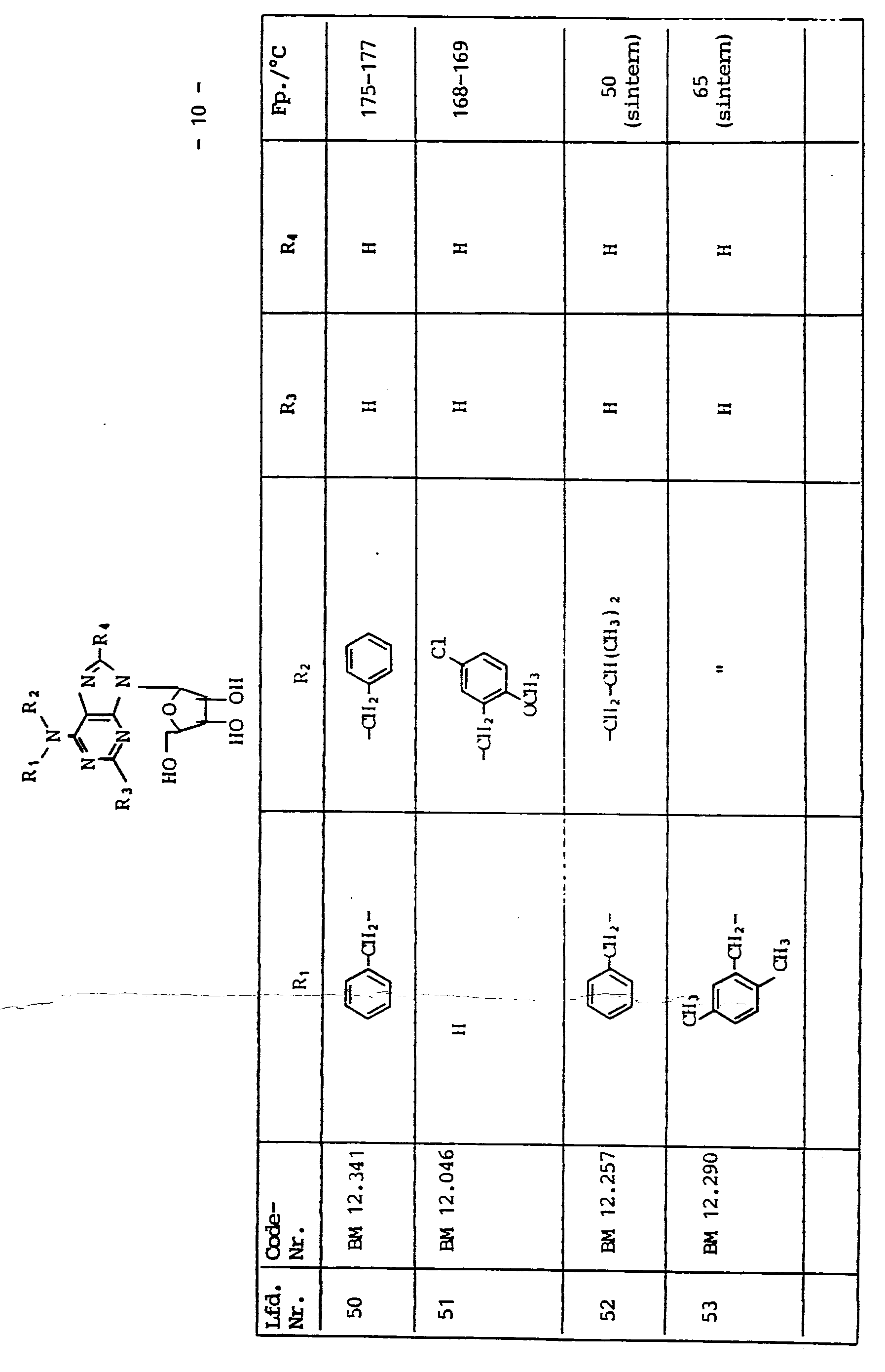

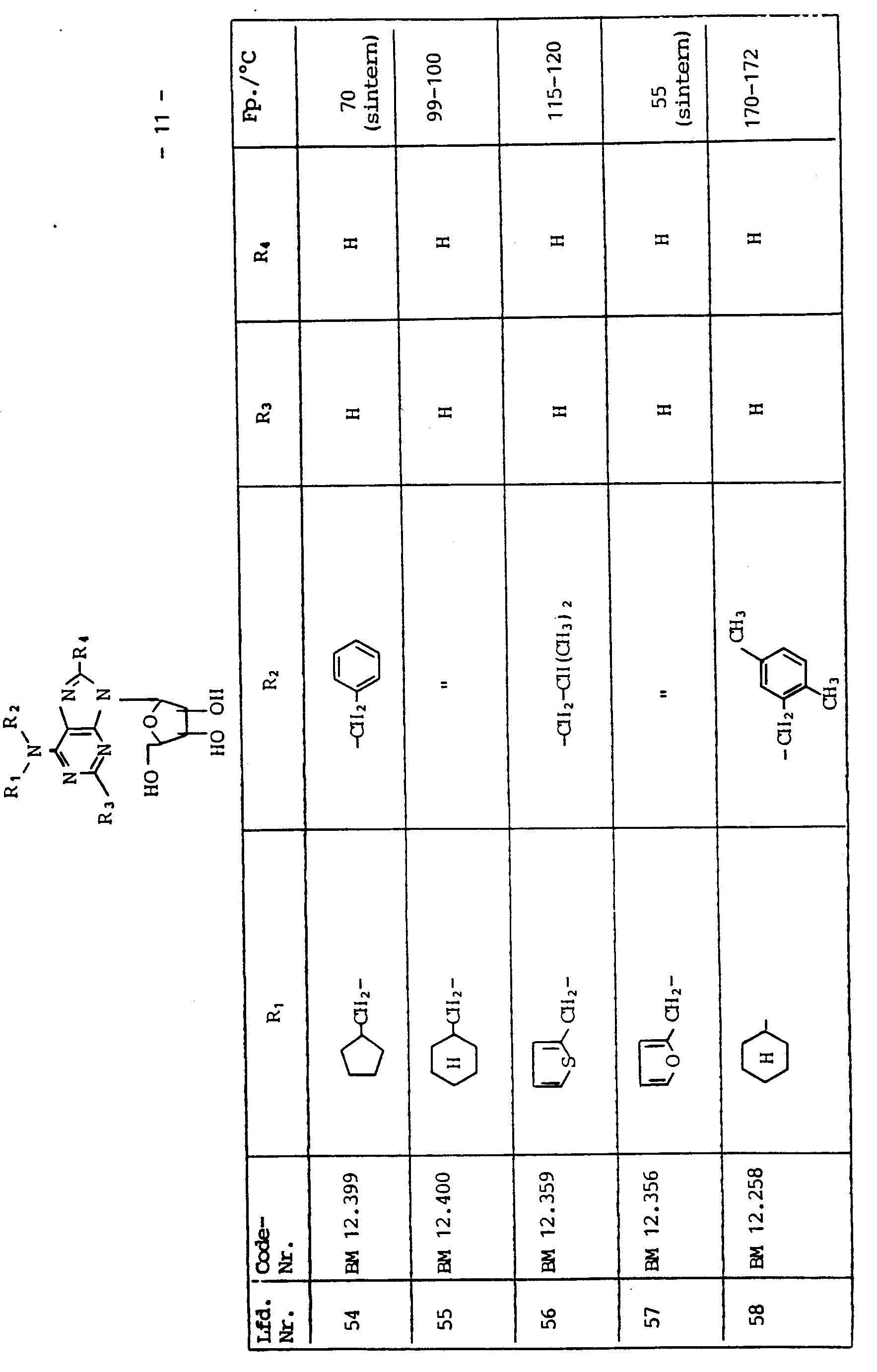

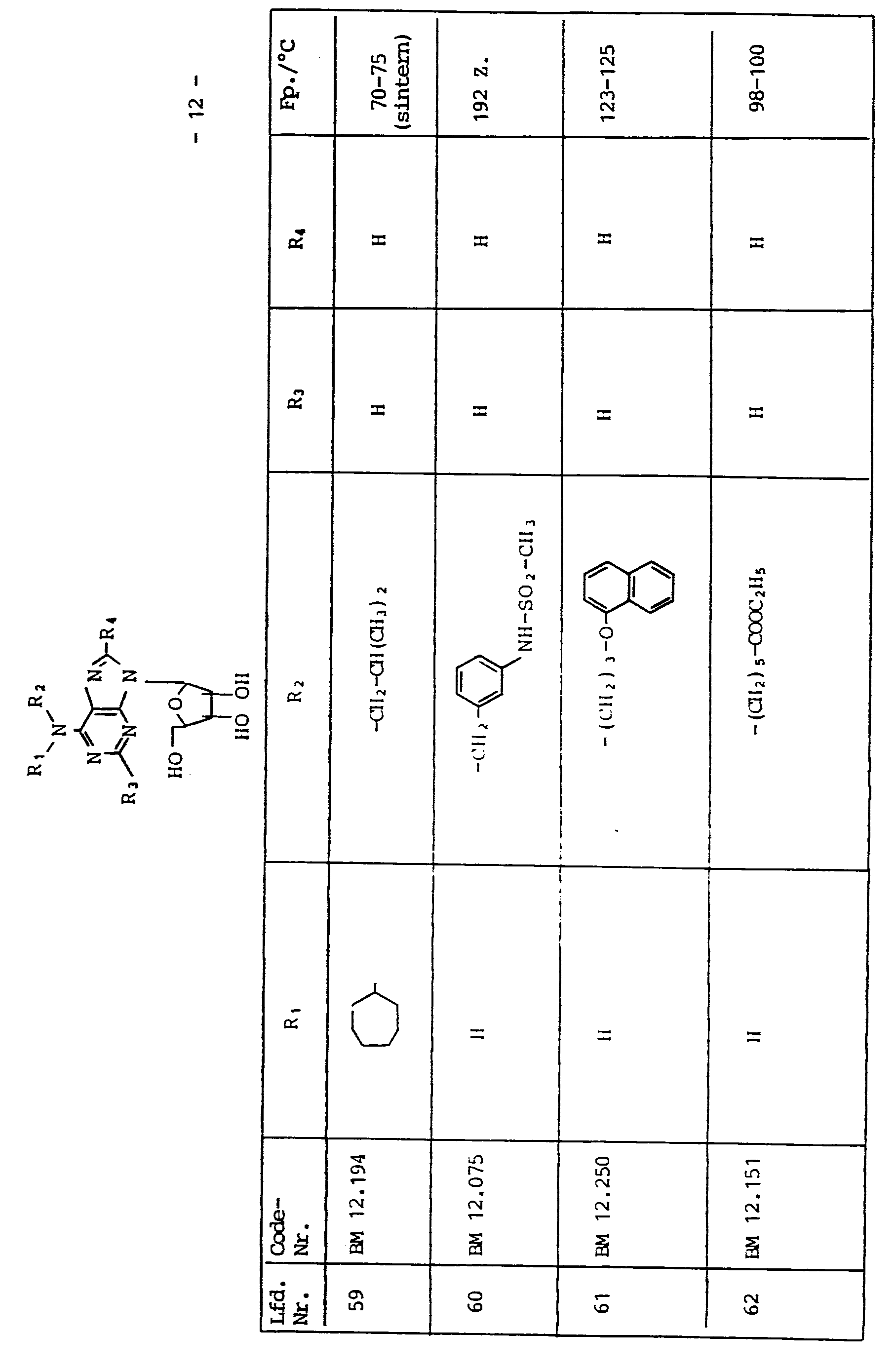

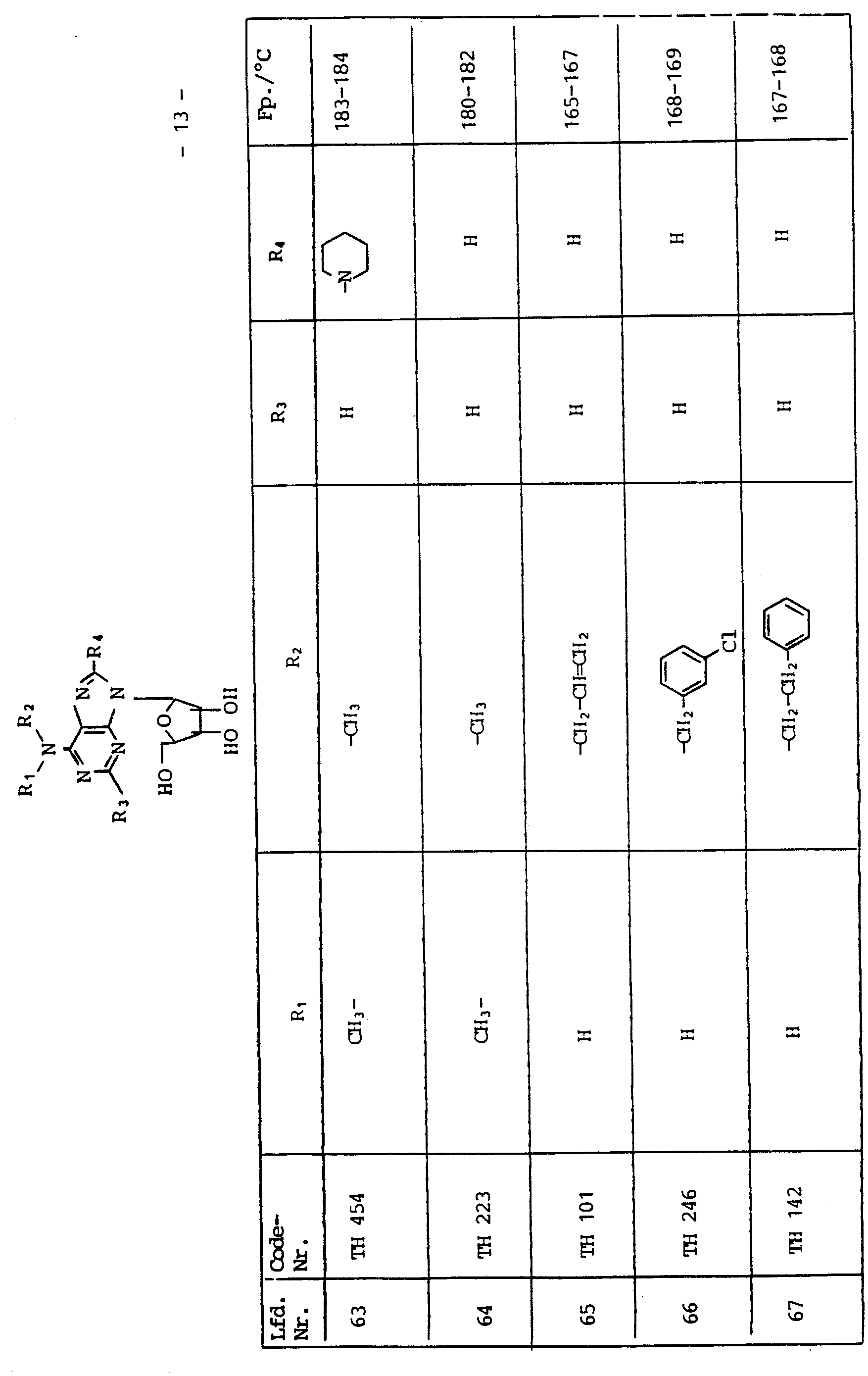

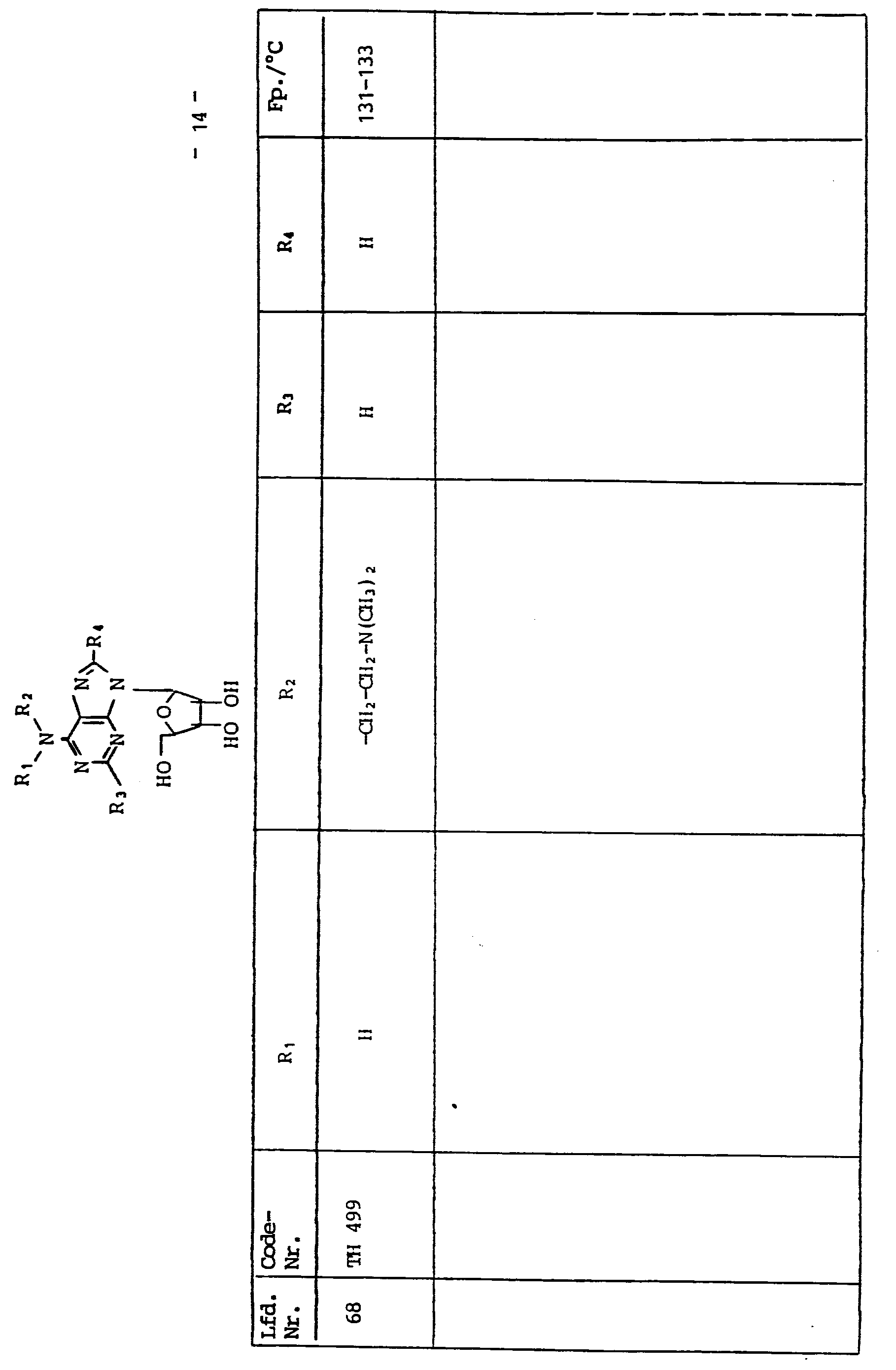

### Einfluß von Xanthin-Derivaten auf die Adenosinwirkung

Es ist bekannt, daß Theophyllin und andere Xanthin-Derivate die typischen Wirkungen des Adenosins und seiner Derivate, z.B. deren Kreislaufeffekte hemmen und aufheben. Es wird angenommen, daß dies auf einer Blockierung der entsprechenden Adenosin-Rezeptoren durch die Xanthin-Derivate beruht, weil diese Verbindungen in der Struktur dem Adenosin ähnlich sind.

Lit.: R.F. Bruns: Biochem. Pharmacol. 30, 325 - 353 (1981); R.F. Bruns et. al.: Proc. Nath Acad Sci. USA, Vo1.80 2077 - 2080 (1983)

Überraschenderweise konnte nun gezeigt werden, daß Theophyllin und andere Xanthin-Derivate zwar die Herzkreislauf-Wirkungen der erfingungsgemäßen Verbindungen der allgemeinen Formel I blockieren, nicht jedoch die antiallergische Wirkung dieser Verbindungen. Im Gegenteil wurde gefunden, daß Theophyllin, welches selbst eine antiallergische Wirksamkeit besitzt, die antiallergische Wirkung der erfindungsgemäßen Substanzen verstärkt. In der folgenden Tabelle 4 wird dies anhand der Beeinflussung der SRA-A-Freisetzung aus Lungengewebe von Meerschweinchen gezeigt, wobei die Untersuchungen analog der vorstehend unter Teil B beschriebenen Methode durchgeführt wurden.

Für die Überprüfung der kombinierten Hemmwirkung von BM 11.189 mit Theophyllin wurde der ungünstige Fall einer besonders starken Antigen-bedingten SRS-A-Freisetzung unter Zusatz von L-PIA gewählt. Bekanntermaßen potenziert L-PIA die Antigen-bedinqte SRS-A-Freisetzunq, wie Adenosin. (vgl. auch Tabelle 1)

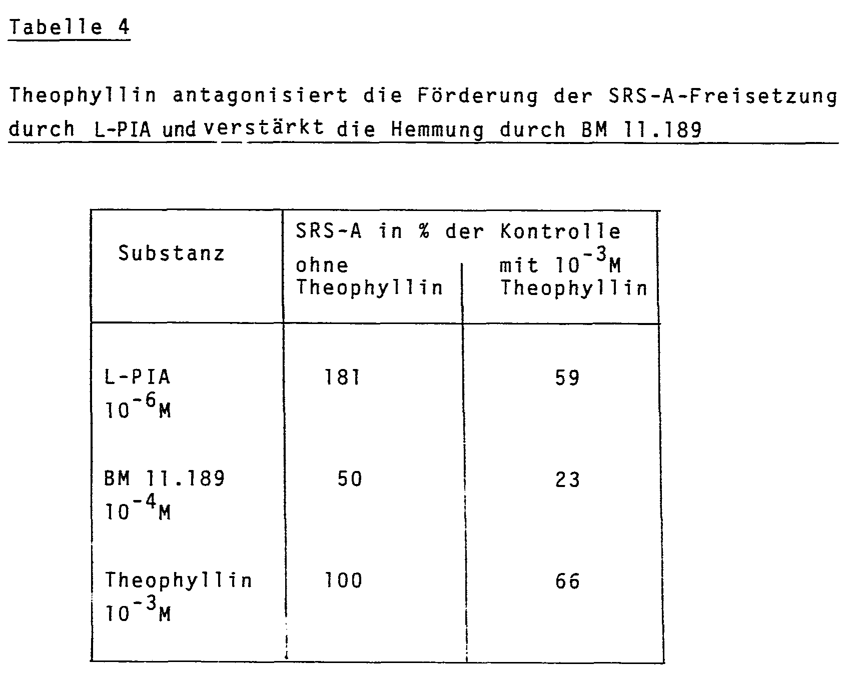

Zum Nachweis der Hemmung der Herzkreislaufwirkungen von Adenosin und den erfindungsgemäßen Adenosin-Derivaten durch Theophyllin und seine Derivate wurde die Hemmung der Erregungsleitung durch Adenosine beim Meerschweinchen und die Aufhebung dieses Effektes durch Theophyllin bestimmt. Gemessen wurde in welcher Dosis Adenosin sowie das Adenosin-Derivat BM 11.189 eine Sinus-Bradykardie oder auch einen AV-Block auslöst und durch welche Theophyllin-Dosierung dieser hemmbar ist.

1. Reduktion der Kammerfrequenz durch BM 11.189 beim narkotisierten Meerschweinchen und Aufhebung dieses Effektes durch Theophyllin

Es war zu klären, ob und in welcher Dosis das Adenosin-Derivat BM 11.189 eine Sinus-Bradykardie oder auch AV-Block auslöst und ob diese Effekte wie bei anderen Adenosin- Derivaten durch Theophyllin hemmbar sind.

### Methode

An Meerschweinchen in Urethan-Narkose wurde die Kammerfrequenz über eine EKG-Ableitung durch Stickelektroden registriert und nach 15 sec bzw. 45 min abgelesen.

Es wurde die Beinflussung der Kammerfrequenz durch steigende, akkumulierende i.v.-Dosen von BM 11.189 untersucht und die Dosis zur Frequenzreduktion auf 120 Schläge/min (DE120) logarithmisch interpoliert.

Weiterhin wurde BM 11.189 mit dieser DE₁₂₀ (entsprechend 6 mg/kg i.v.) allein und unter steigenden akkumulierenden Dosen von Theophyllin (Euphyllin) untersucht.

Ferner wurden 10 mg BM 11.189/kg i.v. zur Erzeugung eines AV-Blocks appliziert und geprüft, ob bzw. wie stark Theophyllin (Euphyllin 20 mg/kg i.v.), prophylaktisch oder nachträglich verabreicht, die Frequenz normalisiert.

### Ergebnisse

Abb. 1 zeigt ein Einzelbeispiel für die Senkung der Kammerfrequenz durch BM 11.189 und den Antagonismus von Theophyllin. In vergleichsweise hoher Dosierung von 10 mg/kg i.v. verursachte BM 11.189 eine extreme Reduktion der Kammerfrequenz, die zum Teil durch eine Abnahme der Sinusfrequenz zustande kam, zum Teil durch einen AV-Block. Vergleichbare Effekte werden durch L-PIA bereits mit Dosierungen zwischen 30 und 100 mcg/kg ausgelöst. Euphyllin hob den AV-Block ganz, die Sinusbradykardie weitgehend auf.

Theophyllin (Euphyllin: 20 mg/kg i.v.) vermochte, sowohl prophylaktisch als auch nachfolgend gegeben, eine durch BM 11.189 (10 mg/kg i.v.) bedingte Frequenz-Veränderung weitgehend im Bereich der Normwerte zu halten oder in diesen zurückzuführen (Tabl. 5).

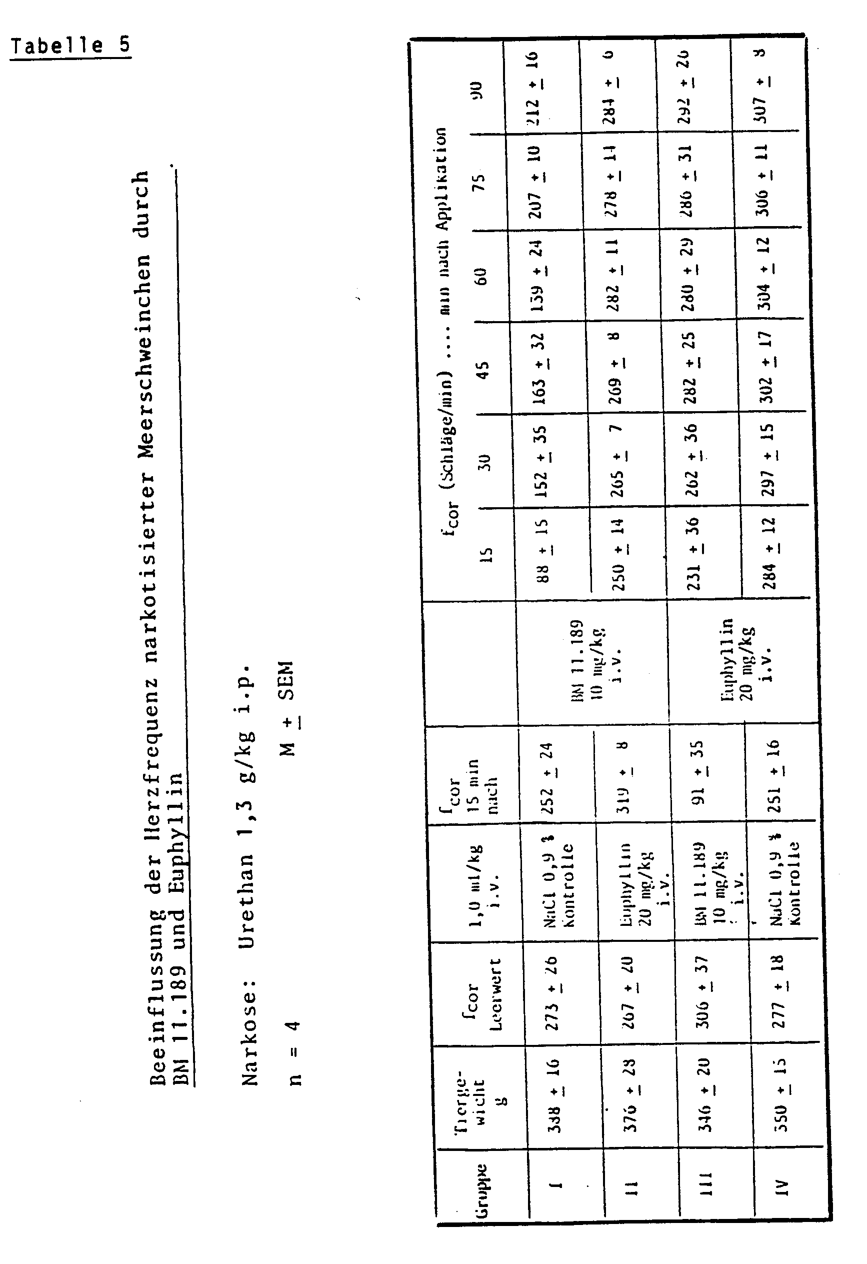

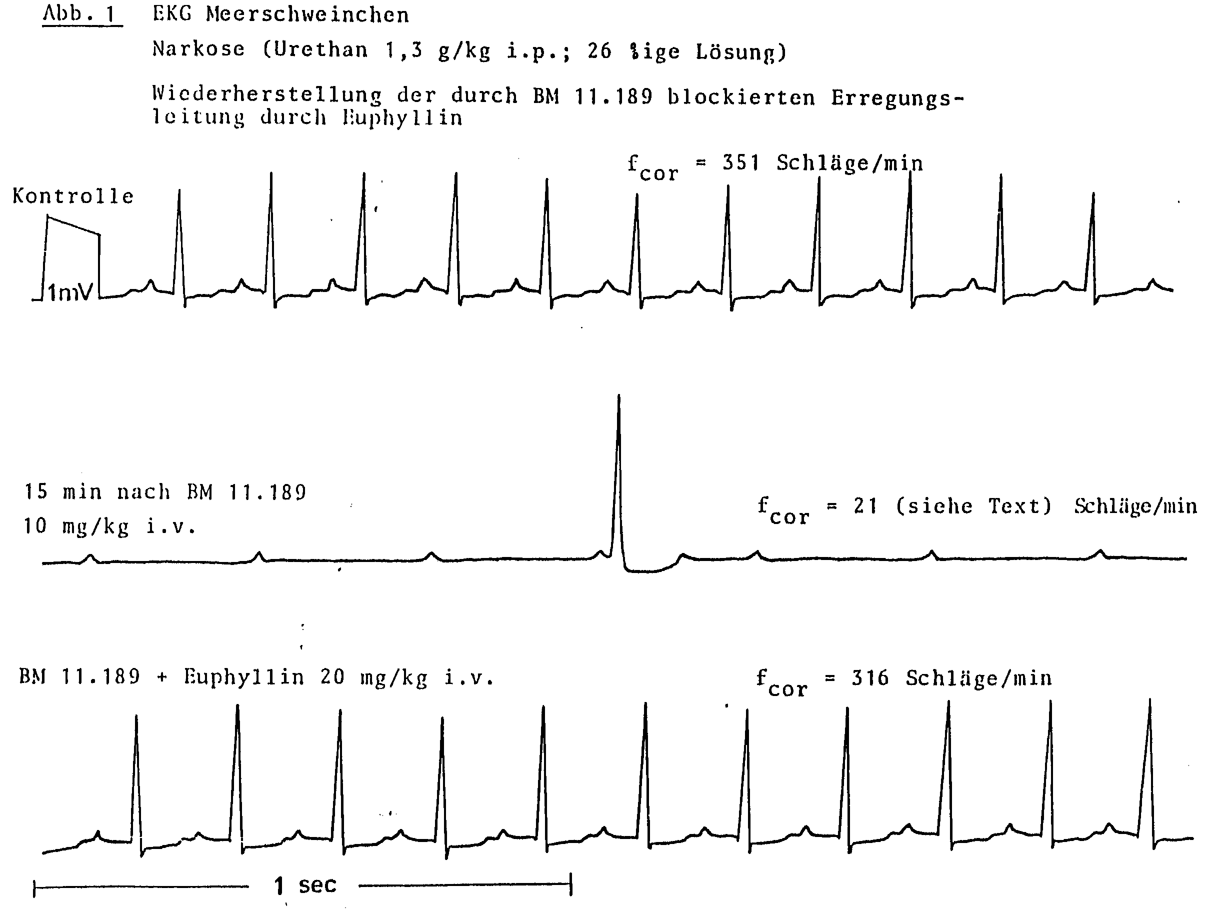

### 2. Antagonismus zwischen Theophyllin-Derivaten und Adenosin

### Methode

Bei Meerschweinchen in Urethan-Narkose wurden in Abständen von 10 min jeweils 0,5 mg/kg Adenosin injiziert. Dieses erzeugt einen atrioventrikulären Block, wodurch die Frequenz der Herzkammer um mehr als 200 Schläge/min abfällt.

Nach 2 Kontrollinjektionen wurden logarithmisch steigende Dosen der zu prüfenden Xanthin-Derivate injiziert, wobei die Gesamtdosis mit jeder Injektion verdoppelt wurde. Die Anfangsdosis betrug bei Euphyllin 1,25 mg/kg, bei den übrigen Substanzen 5 mg/kg. Die Abnahme der Kammerfrequenz unter Adenosin wurde gegen den Logarithmus der Dosis aufgetragen und es wurde die Dosis berechnet, welche die Bradykardie durch Adenosin um 50 % verminderte (DE50).

10 min nach der letzten Injektion von Adenosin wurden 10 mg/kg BM 11.189 injiziert.

### Ergebnisse (Tabelle 6)

Die gewählte Versuchsanordnung machte es möglich, die Wirksamkeit der Theophyllin-Derivate zu quantifizieren. Als Durchschnitt aus 6 Versuchen wurden 9,8 mg/kg Euphyllin benötigt, um die durch Adenosin verursachte Abnahme der Kammerfrequenz auf die Hälfte zu reduzieren. Die höchste Dosis von Euphylli: betrug in 4 Versuchen 20 mg/kg, in den beiden anderen 40 mg/kg. Danach senkte Adenosin die Kammerfrequenz nur noch um 22 Schläge/min.

8-Chloro-theophyllin hemmte die Bradykardie erst in höheren Dosen (ED50: 127 mg/kg; n = 4). Sein Wirkungsmaximum war jedoch nicht niedriger als das von Adenosin. Nach insgesamt 320 mg/kg senkte Adenosin die Kammerfrequenz nur noch um durchschnittlich 11 Schläge/ min.

Theophyllin-Isobutanolamin war etwa halb so wirksam wie Euphyllin.

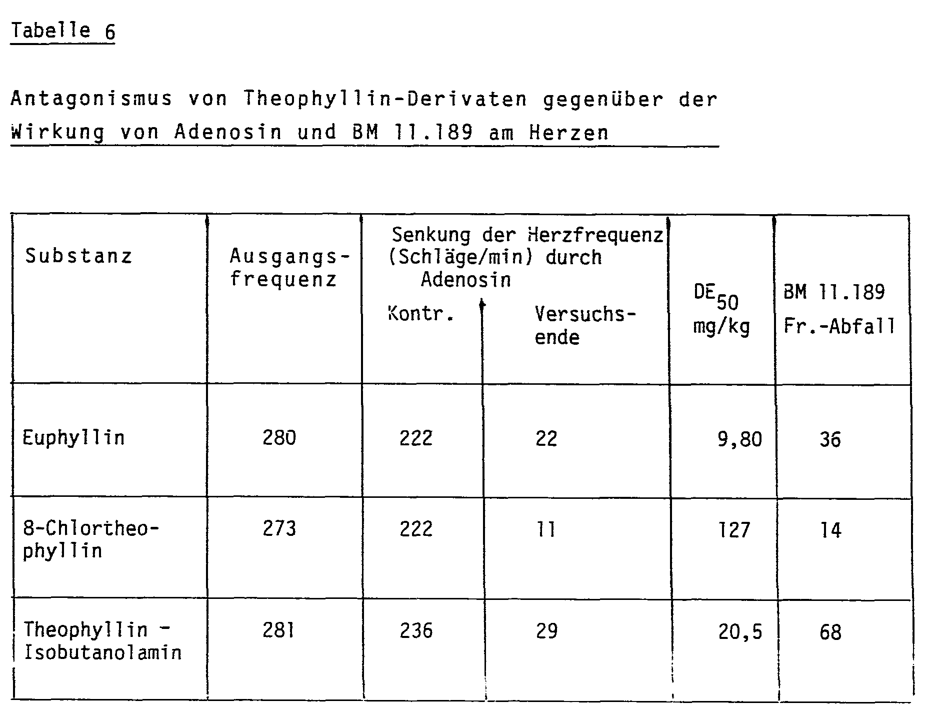

Da die antiallergische Wirkung durch die Zugabe dieser Theophyllin- bzw. Xanthin-Derivate nicht gehemmt sondern im Gegenteil sogar verstärkt wird, weist eine solche Kombination praktisch nur noch die antiallergische Wirkung auf und ist deshalb besonders vorteilhaft zur Behandlung von entzündlichen und allergischen Erkrankungen, z.B. Asthma-Bronchiale.

Gegenstand der vorliegenden Erfindung ist deshalb weiterhin die Verwendung einer Kombination von Adenosin-Derivaten der allgemeinen Formel I und Xanthin-Derivaten der allgemeinen Formel II 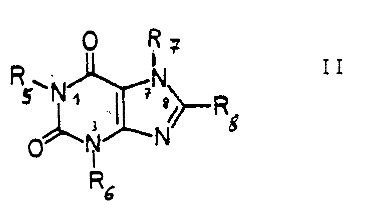 in welcher
R₅ Wasserstoff, eine niedere Alkyl- oder Alkenylgruppe,
R₆ Wasserstoff, eine niedere Alkylgruppe, die durch Hydroxy substituiert sein kann, oder eine niedere Alkenylgruppe, R₇ Wasserstoff oder eine Alkylgruppe, die durch Halogen substituiert sein kann, und
R ₈ Wasserstoff, Halogen, Amino, Nitro, eine niedere Alkylgruppe, die durch Hydroxy, Halogen oder Phenyl substituiert sein kann, eine niedere Alkylthio- oder Alkoxygruppe, eine mono- oder dialkylierte Aminogruppe, eine Cycloalkylgruppe oder eine Arylgruppe, die ein- oder mehrfach durch Halogen, Alkyl, Alkoxy, Nitro, Amino, Carboxy,Hydroxy oder mono- oder dialkyliertes Amino substituiert sein kann,
   bedeuten,

zur Behandlung allergischer Erkrankungen sowie von entzündungsbedingten bronchospastischen und bronchokonstriktorischen Reaktionen.

Die Verbindungen der allgemeinen Formel II sind bekannte Verbindungen

Unter einer niederen Alkylgruppe der Substituenten R₅, R₆, R₇ und R₈ sind Reste mit 1 - 6 Kohlenstoffatome, vorzugsweise Methyl, Ethyl, n-Propyl, Isopropyl, n-Hexyl zu verstehen.

Eine niedere Alkylthiogruppe enthält 1 - 6 Kohlenstoffatome, bevorzugt ist die Methylthiogruppe.

Eine niedere Alkoxygruppe enthält 1 - 6 Kohlenstoffatome, bevorzugt sind die Methoxy- und Ethoxygruppen.

Eine niedere Alkenylgruppe enthält 2 - 6 Kohlenstoffatome, bevorzugt ist die Allylgruppe.

Unter Halogen sind Fluor, Chlor. Brom und Jod zu verstenen, bevorzugt sind Fluor, Chlor und Brom.

Ein Arylrest der Substituenten R₈ mit Phenyl- oder Naphthyl, wobei der Phenylrest bevorzugt ist.

Bevorzugte Xanthin-Derivate der Formel II sind Verbindungen in denen R₅ Wasserstoff, oder Cₗ - C4 Alkyl, R₆ Wasserstoff oder Cₗ - C4 Alkyl, R₇ Wasserstoff oder C₁ - C₄ Alkyl,R₈ wasserstoff, Halogen, C₁ - C4 Alkyl oder eine gegebenenfalls substituierte Phenylgruppe bedeuten, insbesondere die Verbindung Theophyllin und 8-Chlortheophyllin.

Weiterer Gegenstand der Erfindung sind daher auch Arzneimittel, die Verbindungen der allgemeinen Formel I bzw. I' neben üblichen Trägerstoffen enthalten.

Zur Herstellung pharmazeutischer Mittel werden die Verbindungen der allgemeinen Formel I bzw. I' in an sich bekannter Weise mit geeigneten pharmazeutischen Trägersubstanzen gemischt, gegebenenfalls granuliert und beispielsweise zu Tabletten oder Drageekernen verpreßt. Ebenso ist eine Abfüllung der Mischung in Steckkapseln möglich. Unter Zugabe entsprechender Hilfsstoffe kann auch eine Lösung oder Suspension in Wasser, Öl (z.B. Olivenöl) oder hochmolekularen Polymeren (z.B. Polyethylenglykol) hergestellt und zu Injektionslösungen, Weichgelatinekapseln, Saft oder Tropfen verarbeitet werden.

Die Substanzen der allgemeinen Formel 1 bzw. I' können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositaetsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Geschmacks- und Süßstoffe enthalten. Für die äußere Anwendung können die erfindungsgemäßen Substanzen der Formel I bzw. I' auch in Form von Pudern und Salben verwendet werden. Sie werden dazu z. B. mit pulverförmigen physiologisch verträglichen Verdünnungsmittel bzw. üblichen Salbengrundlagen vermischt.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis/Erwachsenem 120 mg pro Adenosin-Derivat und bis 60 mg pro Xanthin-Derivat. Normalerweise sind Einzeldosen von 10 bis 40 und vorzugsweise 30 bis 120 mg/Tag in einer oder mehreren Anwendungen pro Tag wirksam, um die gewünschten Resultate zu erhalten.

Ferner sind Gegenstand der Erfindung Arzneimittelkombinationen, die neben üblichen Träger- und Hilfsstoffen als Wirkkomponente eine Verbindung der allgemeinen Formel I bzw. I' und eine Verbindung der allgemeinen Formel II enthalten. Für diese Kombinationen kommen im allgemeinen die gleichen Zubereitungsformen in Betracht, wie für die oben aufgeführten Einzelsubstanzen. Die beiden Wirkstoffe, ein Adenosin-Derivat der Formel I bzw. I' sowie das Xanthin-Derivat der Formal II liegen in der Regel in der Zubereitungsform im Verhältnis 2:1 bis 1:20 vor, vorzugsweise 1:10.

Eine geeignete Zubereitungsform besteht aus 10 - 20 mg BM 11.189 als Adenosin-Derivat und 235 mg Euphillin als Xanthin-Derivat, sowie geeigneten Trägerstoffen und wird in Form von 300 mg Tabletten hergestellt, die in der Regel dreimal pro Tag oral eingenommen werden.

## Patentansprüche

1. Verwendung von Adenosin-Derivaten der allgemeinen Formel I 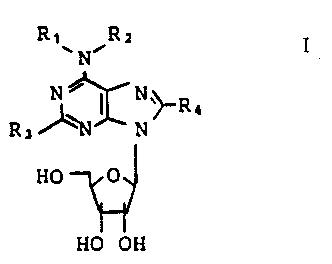 in welcher R₁ und R₂ gleich oder verschieden sind und eine geradkettige oder verzweigte Alkylgruppe, die ein- oder mehrfach durch Hydroxy-, Alkoxycarbonyl-oder Alkoxy-Gruppen, durch Phenalkyl oder Alkyl substituierte Aminogruppen, wobei der Pnenylteil gegebenenfalls ebenfalls substitulert sein kann, durch gegebenenfalls ein- oder mehrfach durch Hydroxy, Alkoxy, Alkyl, Trifluormethyl, Halogen, Amino oder Methylsulfonamino substituierte Aryl-, Hetaryl-oder Aryloxy-Gruppen oder durch Cycloalkyl, das mit einem Arylring anelliert sein kann, substituiert sein kann. eine geradkettige oder verzweigte Alkenylgruppe, eine gesättigte oder ungesättigte Cycloalkylgruppe, die mit einem Arylring anelliert sein kann, oder gemeinsam mit dem Stickstoffatom, an das sie gebunden sind, einen heterocyclischen Ring bilden können, der gegebenenfalls durch ein weiteres Sauerstoff-, Schwefel- oder Stickstoffatom unterbrochen sein kann, wobei der Stickstoff durch Alkyl oder Aralkyl substituiert sein kann,
R₃ Wasserstoff, Hydroxy, Alkoxy, Halogen, Morpholin, Piperidin oder eine Aminogruppe, die durch Alkyl oder gegebenenfalls substituiertes Aralkyl substituiert sein kann, und
R₄ Wasserstoff, Halogen, Morpholin, Piperidin oder eine gegebenenfalls durch Alkyl oder Aralkyl substituierte Aminogruppe darstellen,
wobei einer der Reste R₁ oder R₂ auch Wasserstoff sein kann, mit der Maßgabe, daß der andere Rest nicht eine Phenylisopropylgruppe sein darf, sowie deren pharmakologisch verträglichen Salze ,
zur Behandlung allergischer Erkrankungen sowie für entzündungsbedingte bronchospastische und brochokonstriktorische Reaktionen.

2. Verwendung von Adenosin-Derivaten gemäß Anspruch 1, in denen R₁ und R₂ die angegebene Bedeutung haben und R₃ und R₄ Wasserstoff darstellen.

3. Verwendung von Adenosin-Derivaten gemäß Anspruch 2, in denen R₁ und R₂, R₃ und R₄ die angegebene Bedeutung haben, mit der Maßgabe, ^{daß} R₁ und R₂ nicht Wasserstoff bedeuten.

4. Adenosin-Derivate der allgemeinen Formel I' 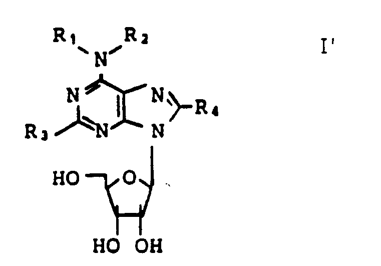 in welcher
R₁ und R₂ gleich oder verschieden sind und Wasserstoff eine geradkettige oder verzweigte Alkylgruppe, die ein- oder mehrfach durch Hydroxy-, Alkoxycarbonyl-oder Alkoxy-Gruppen,durch Phenalkyl oder Alkyl substituierte Aminogruppen, wobei der Phenylteil gegebenenfalls ebenfalls substituiert sein kann, durch gegebenenfalls ein- oder mehrfach durch Hydroxy, Alkoxy, Alkyl, Trifluormethyl, Halogen, Amino oder Methylsulfonamino substituierte Aryl-, Hetaryl-oder Aryloxy-Gruppen oder durch Cycloalkyl, das mit einem Arylring anelliert sein kann, substituiert sein kann
eine geradkettige oder verzweigte Alkenylgruppe, eine gesättigte oder ungesättigte Cycloalkylgruppe, die mit einem Arylring anelliert sein kann, oder gemeinsam mit dem Stickstoff, an das sie gebunden sind, einen heterocyclischen Ring bilden können, der gegebenenfalls durch ein weiteres Sauerstoff-, Schwefel- oder Stickstoffatom unterbrochen sein kann, wobei der Stickstoff durch Alkyl oder Aralkyl substituiert sein kann,
R₃ Hydroxy, Alkoxy, Halogen, Morpholin, Piperidin oder eine Aminogruppe, die durch Alkyl oder gegebenenfalls substituiertes Aralkyl substituiert sein kann, und
R₄ Halogen, Morpholin, Piperidin oder eine gegebenenfalls durch Alkyl oder Aralkyl substituierte Aminogruppe darstellen, wobei einer der Reste R₃ oder R₄ auch Wasserstoff sein kann, sowie deren pharmakologisch verträglichen Salze.
. Verbindungen gemäß Anspruch 4, in denen
R₁ Wasserstoff, C₁- C8 Niederalkyl oder Hydroxyethyl,
R₂ Wasserstoff, C₁- C8 Niederalkyl, Benzyl, Hydroxyethyl oder Cyclohexyl,
R₁ und R₂ zusammen mit dem Stickstoffatom einen Piperidin- oder Morpholin-Ring bilden,
R₃ Wasserstoff, eine Amino-, eine Benzylamino-, die gegebenenfalls durch eine Methylgruppe substituiert ist, eine Morpholino- oder Piperidino-Gruppe
R₄ eine Benzylamino-, Morpholino- oder Piperidino-Gruppe bedeuten, wobei für den Fall, daß R₃ eine Morpholino- oder Piperidino darstellt, R₄ auch Wasserstoff sein kann

6. Verwendung von Adenosin-Derivaten der allgemeinen Formel I gemäß Ansprüche 1, 2 oder 3 zusammen mit Xanthin-Derivaten der allgemeinen Formel II 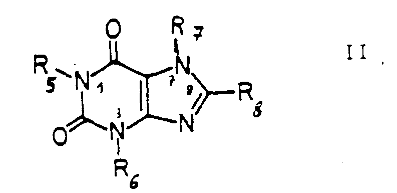 in welcher
R₅ Wasserstoff, eine niedere Alkyl- oder Alkenylgruppe,
R₆ Wasserstoff, eine niedere Alkylgruppe, die durch Hydroxy substituiert sein kann, oder eine niedere Alkenylgruppe
R₇ Wasserstoff oder eine niedere Alkylgruppe, die durch Halogen substituiert sein kann, und
R₈ Wasserstoff, Halogen, Amino, Nitro, eine niedere Alkylgruppe, die durch Hydroxy, Halogen oder Phenyl substituiert sein kann, eine niedere Alkylthio- oder Alkoxygruppe, eine mono- oder dialkylierte Aminogruppe, eine Cycloalkylgruppe oder eine Arylgruppe, die ein- oder mehrfach durch Halogen, Alkyl, Alkoxy, Nitro, Amino, Carboxy, Hydroxy oder mono- oder dialkyliertes Amino substituiert sein kann, bedeuten,
zur Behandlung von allergischen Erkrankungen sowie von entzündungsbedingten bronchospastischen und bronchokonstriktorischen Reaktionen.

7. Arzneimittel zur Behandlung allergischer Erkrankungen sowie von entzündungsbedingten bronchospastischen und bronchokonstriktorischen Reaktionen, enthaltend eine Verbindung der allgemeinen Formel I gemäß Ansprüche 1, 2 oder 3.

8. Arzneimittel zur Behandlung allergischer Erkrankungen sowie von entzündungsbedingten bronchospastischen und bronchokonstriktorischen Reaktionen, enthaltend ein Adenosin-Derivat der allgemeinen Formel I gemäß Ansprüche 1, 2 oder 3 und ein Xanthin-Derivat der allgemeinen Formel II gemäß Anspruch 6.

9. Arzneimittel zur Behandlung allergischer Erkrankungen sowie von entzündungsbedingten bronchospastischen und bronchokonstriktorischen Reaktionen, enthaltend eine Verbindung der allgemeinen Formel I' gemäß Ansprüche 4 oder 5.
